# FASCICULE DE BREVET EUROPEEN

(11) **EP 2 054 439 B1**
(45) Date de publication et mention de la délivrance du brevet: **01.06.2011**
(21) Numéro de dépôt: 07802491.6
(22) Date de dépôt: 03.08.2007
(51) Int. Cl.: C07K 14/705, A61K 38/16

(54) **PEPTIDES DERIVES DU RÉCEPTEUR CD4 ET LEUR PROCÉDÉ DE PRÉPARATION**
VOM CD4-REZEPTOR ABGELEITETE PEPTIDE UND VERFAHREN ZUR HERSTELLUNG DAVON
CD4-RECEPTOR-DERIVED PEPTIDES AND METHOD FOR THE PREPARATION THEREOF

(30) Priorité: 04.08.2006 FR 0607149
(43) Date de publication de la demande: 06.05.2009
(73) Titulaire: INSTITUT PASTEUR, 75015 Paris (FR); CENTRE NATIONAL DE LA RECHERCHE SCIENTIFIQUE (CNRS), 75016 Paris (FR)
(72) Inventeur: BALEUX, Françoise, 75005 Paris (FR); BONNAFFE, David, 75011 Paris (FR); LORTAT-JACOB, Hugues, 38330 Saint Ismer (FR)
(74) Mandataire: Warcoin, Jacques
(86) Numéro de dépôt international: PCT/EP2007/058069
(87) Numéro de publication internationale: WO 2008/015273

(56) Documents cités:
- WO-A2-02/059146
- WO-A2-03/089000
- WO-A2-2005/003296
- US-A1- 2005 176 642
- STRICHER F ET AL.: "A high-throughput fluorescence polarization assay specific to the CD4 binding site of HIV-1 glycoproteins based on a fluorescein-labelled CD4 mimic" BIOCHEMICAL JOURNAL, vol. 390, 2005, pages 29-39, XP002419847
- HUANG C C ET AL: "Scorpion-Toxin Mimics of CD4 in Complex with Human Immunodeficiency Virus gp120" STRUCTURE, CURRENT BIOLOGY LTD., PHILADELPHIA, PA, US, vol. 13, no. 5, mai 2005 (2005-05), pages 755-768, XP004910457 ISSN: 0969-2126
- MARTIN L ET AL: "Rational design of a CD4 mimic that inhibits HIV-1 entry and exposes cryptic neutralization epitopes" NATURE BIOTECHNOLOGY, NATURE PUBLISHING GROUP, NEW YORK, NY, US, vol. 21, no. 1, janvier 2003 (2003-01), pages 71-76, XP001156366 ISSN: 1087-0156

### LISTE DE SEQUENCES

<110> INSTITUT PASTEUR
   CENTRE NATIONAL DE LA RECHERCHE SCIENTIFIQUE (CNRS) BALEUX Françoise
<120> Nouveaux peptides activés, purifiés et isolés, dérivés du récepteur CD4 (mini-CD4) et leur procédé de préparation
<130> D24490
<150> FR0607149
   <151> 2006-08-04
<160> 4
<170> PatentIn version 3.3
<210> 1
   <211> 26
   <212> PRT
   <213> Séquence artificielle
<220>
   <223> Peptide dérivé du CD4
<220>
   <221> MOD RES
   <222> (1)..(1)
   <223> TPA-Asn
<220>
   <221> misc_feature
   <222> (22)..(22)
   <223> Xaa représente une Bi-phénylalanine
<220>
   <221> MOD_RES
   <222> (26) .. (26)
   <223> Val-NH₂
<400> 1
   Asn Leu His Lys Cys Gln Leu Arg Cys Ser Ser Leu Gly Leu Leu Gly
   1               5                   10                  15
   Arg Cys Ala Gly Ser Xaa Cys Ala Cys Val
               20                  25
<210> 2
   <211> 26
   <212> PRT
   <213> Séquence artificielle
<220>
   <223> Peptide dérivé du CD4
<220>
   <221> MOD_RES
   <222> (1)..(1)
   <223> TPA-Asn
<220>
   <221> misc_feature
   <222> (22) .. (22)
   <223> Xaa représente une Bi-phénylalanine
<400> 2
   Asn Leu His Lys Cys Gln Leu Arg Cys Ser Ser Leu Gly Leu Leu Gly
   1               5                   10                  15
   Arg Cys Ala Gly Ser Xaa Cys Ala Cys Val
               20                  25
<210> 3
   <211> 27
   <212> PRT
   <213> Séquence artificielle
<220>
   <223> Peptide GPR1
<220>
   <221> MOD_RES
   <222> (27)..(27)
   <223> Cys-NH₂
<400> 3
   Met Glu Asp Leu Glu Glu Thr Leu Phe Glu Glu Phe Glu Asn Tyr Ser
   1               5                   10                  15
   Tyr Asp Leu Asp Tyr Tyr Ser Leu Glu Ser Cys
               20                  25
<210> 4
   <211> 27
   <212> PRT
   <213> Séquence artificielle
<220>
   <223> Peptide GPR1
<400> 4
   Met Glu Asp Leu Glu Glu Thr Leu Phe Glu Glu Phe Glu Asn Tyr Ser
   1               5                   10                  15
   Tyr Asp Leu Asp Tyr Tyr Ser Leu Glu Ser Cys
               20                  25

## Description

La présente invention a pour objet un peptide activé dérivé du récepteur du CD4, qui est capable de se coupler par liaison covalente à une molécule organique et permet ainsi de générer de multiples dérivés potentiellement antiviraux. Un autre objet est une molécule conjuguée comprenant le peptide dérivé du récepteur CD4 et une molécule organique, de préférence le peptide GPR1 ou un polyanion. Une telle molécule conjuguée peut notamment être utilisée dans les traitements antiviraux, notamment le traitement du sida. L'invention a encore pour objet les procédés de préparation du peptide activé dérivé du récepteur CD4 et de la molécule conjuguée.

Les trithérapies, associant inhibiteurs nucléosidiques (INTI), non nucléosidiques (INNTI) et/ou antiprotéases (IP), permettent une réduction de la charge virale en dessous des seuils de détectabilité chez un grand nombre de patients séropositifs au VIH. Cette efficacité a permis une diminution très notable de la mortalité liée à l'infection à VIH. Malheureusement, des génotypes indiquant une résistance à un antiviral ont été trouvés chez 80 % des patients et, de manière plus inquiétante, 45,5 % des populations virales sont résistantes à une association 1NTI/IP et 26 % à une association de trois classes d'anti-VIH (Tamalet et al., AIDS. 2003 Nov 7;17(16):2383-8). Ce constat est particulièrement préoccupant lorsque l'on observe des effets secondaires liés à une prise au long terme des trithérapies (lipoatrophies, lipodystrophies, hypertriglycidémie, hypercholestérolémie, neuropathies...), observés chez 70 % des patients sous traitement, entraînant une mauvaise observance et des arrêts « sauvages » souvent à l'origine de l'apparition de résistances. La mise au point de traitements moins contraignants, entraînant moins d'effets secondaires et ne présentant pas de profil de résistances croisées reste donc une priorité, malgré le grand nombre de médicaments actuellement commercialisés. Dans ce but, il est indispensable de cibler d'autres étapes du cycle réplicatif du VIH que la rétrotranscription et la protéolyse.

La découverte des corécepteurs CXCR4 et CCR5 (Broder CC & Collman RG, J Leukoc Biol. 1997 Jul;62(1):20-9. Review) a ouvert la voie à la compréhension des mécanismes d'infection d'une cellule hôte. La première étape implique un attachement du VIH à la surface cellulaire, grâce à une interaction entre la glycoprotéine gp120 du VIH et le récepteur CD4 de la cellule cible. Il s'en suit un changement conformationnel de la gp120 qui va exposer un épitope, dit CD4i (pour CD4 induit) auparavant masqué, qui constitue une partie du site de liaison aux corécepteurs. L'interaction gp120/corécepteur va provoquer un nouveau changement conformationnel permettant l'exposition de la gp41, ce qui initie le processus de fusion des membranes. La compréhension de ce mécanisme a permis la mise au point de nouveaux types de médicaments inhibant soit l'interaction gp120/CD4 (Bristol-Myers Squibb BMS-488043 en phase clinique IIa), soit l'interaction gp120/CCR5 (Schering-Plough SCH-D et Pfizer UK-427,657 en phase III en 2004-05, et GlaxoSmithKline GSK GW873140/AK602 en phase I), soit la phase de fusion en se liant à la gp41 (T20, Fuzéon^{®}, Roche).

Ces résultats extrêmement positifs attestent que les approches visant à bloquer l'une des étapes de la pénétration du VIH dans une cellule sont pertinentes. On peut cependant remarquer que parmi les inhibiteurs de l'interaction gpl20/corécepteur en développement clinique, seuls des produits inhibant l'interaction avec CCR5 sont étudiés. Les composés se liant à CXCR4 ont vu leurs développements arrêtés, comme l'AMD8664 qui provoque des effets secondaires au niveau cardiovasculaire liés à son mode d'action (Gao Z & Metz WA, 2003 Sep;103(9):3733-52). L'absence de traitement inhibant l'interaction gp120/CXCR4 pourrait conduire à terme à l'échec de ceux visant CCR5 en provoquant une évolution de la population virale vers un tropisme X4 (VIH infectant via CXCR4), souvent associée à une accélération de la déplétion en lymphocytes T CD4+. Curieusement, le site de liaison aux corécepteurs, hautement conservé chez le VIH-1, le VIH-2 et le SIV (Rizzuto CD et al, 1998 Jun 19;280(5371):1949-53 ; Kwong PD et al., Nature. 1998 Jun 18;393(6686):648-59), ne semble pas être une cible thérapeutique sur laquelle beaucoup de recherches sont menées. Le fait que ce site soit caché, tant que la gp120 n'est pas liée à CD4, rend en effet l'accès de molécules à ce site difficile. La présente invention propose de résoudre ce problème en préparant, par synthèse chimique, des composés capables de se lier au site conservé de liaison aux corécepteurs ainsi qu'à la boucle V3. De tels composés seraient a priori capables d'inhiber l'interaction gp120/CD4 et l'interaction gp120/corécepteur et ce, quelque soit le type de souche de VIH.

Il est connu depuis de nombreuses années que certains polyanions, comme l'héparine (HP) ou le sulfate de dextran (DS), mais pas le chondroïtine sulfate (CS), sont capables d'inhiber l'infection de cellules par le VIH (Esté JA, et al., Mol Pharmacol. 1997 Jul;52(1):98-104). Ils ne sont cependant pas utilisés en clinique notamment à cause de leurs effets anticoagulants (Flexner C et al., Antimicrob Agents Chemother. 1991 Dec;35(12):2544-50). Récemment il a été montré que le mécanisme moléculaire de cette inhibition était lié à une interaction du polyanion avec la boucle V3 (Moulard M et al., J Virol. 2000 Feb;74(4):1948-60.

D'autre part, différentes études ont exploré l'utilisation de CD4 soluble pour inhiber l'interaction du virus avec le CD4 exprimé à la surface des cellules cibles du VIH. Cette solution s'est révélée inefficace, parce qu'en se fixant aux virus, le CD4 soluble expose l'épitope CD4i, et favorise en fait l'interaction du virus au co-récepteur CCR5 ou CXCR4, ce qui dans certains cas augmente l'infection (Schenten D. et al, 1999, J Virol.73 : 5373-80).

Il est connu de la demande de brevet internationale publiée sous le numéro WO 03/089000 qu'un peptide dérivé du récepteur CD4, lorsqu'il est mis en présence avec un polyanion, possède une action anti-VIH. Il est notamment préconisé que des composés dans lesquels le peptide et le polyanion sont liés peuvent être préparés selon la description faite dans l'article Najjam S. et al. (Cytokine 1997, 9(12): 1013-1022) (cf. point 1.1 de la partie EXEMPLES).

Dans la présente invention, les inventeurs ont obtenu des peptides activés dérivés du récepteur CD4, susceptibles de lier directement et de manière covalente le polyanion ou toute autre molécule organique susceptible de jouer un rôle dans l'action anti-VIH avec le miniCD4. Cette activation requiert l'introduction préalable de résidus d'acides aminés particuliers dans le peptide natif. En particulier, les inventeurs ont découvert que la présence d'un et un seul résidu d'acide aminé lysine dans la séquence du peptide dérivé du récepteur CD4 est indispensable pour obtenir un peptide activé selon l'invention. En outre, cet unique résidu d'acide aminé lysine doit être en une position bien définie dans la séquence du peptide dérivé du récepteur CD4. La conception du peptide miniCD4 comprenant un seul résidu d'acide aminé lysine en position définie permet d'introduire sélectivement et directement n'importe quelle fonction sur le miniCD4.

La présente invention offre ainsi des composés activés permettant de générer de multiples dérivés potentiellement antiviraux. Ces dérivés consistent en des molécules conjuguées comprenant un peptide CD4 couplé de manière spécifique à une molécule organique, telle qu'un polyanion, au moyen d'un bras espaceur.

Cette approche est avantageuse du point de vue thérapeutique pour inhiber l'attachement du virus sur les cellules, car elle vise directement le virus et non les cellules elles mêmes. Elle est donc, au premier abord, dépourvue des effets cellulaires qui sont observés avec les médicaments se liant aux corécepteurs. D'autre part, au vu de la conservation des sites impliqués en fonction des différents tropismes viraux, les composés selon l'invention devraient interagir avec les gp120 de différents isolats viraux. De plus, même s'il est illusoire de penser que des résistances n'apparaîtront pas, ce nouveau type de composé ne devrait provoquer que difficilement leur émergence. En effet, le site CD4 de gp120 doit rester intègre pour continuer à se lier à CD4 et les résidus basiques impliqués dans la liaison au polyanion le sont aussi pour l'interaction avec les corécepteurs, et une mutation dans l'un de ces deux sites conduirait à un virus peu fonctionnel. Finalement, dans le cadre de la présente invention, une version entièrement synthétique des composés peut être développée, garantissant ainsi une préparation en quantité importante, homogène, et parfaitement définie. La méthode de couplage est simple, rapide et quantitative.

Ainsi, selon un premier aspect, la présente invention a pour objet un procédé de préparation d'un peptide activé dérivé du récepteur CD4, ledit peptide, une fois activé, étant capable de se coupler par liaison covalente à une molécule organique, et dans lequel ledit peptide dérivé du récepteur CD4 comprend ou est constitué par la séquence générale (I) suivante :

Xaa^{f} - P1-Lys- Cys - P2 - Cys - P3-Cys--Xaa^{g}-Xaa^{h}-Xaaⁱ-Xaa^{j}-Cys-Xaa^{k}-Cys-Xaa^{l}-Xaa^{m}, (I)

dans laquelle :
- P1 représente 3 à 6 résidus d'acides aminés,
- P2 représente 2 à 4 résidus d'acides aminés,
- P3 représente 6 à 10 résidus d'acides aminés,
- Xaa^{f} représente la N-acétylcystéïne (Ac-Cys) ou l'acide thiopropionique (TPA),
- Xaa^{g} représente Ala ou Gln,
- Xaa^{h} représente Gly ou (D)Asp ou Ser,
- Xaaⁱ représente Ser ou His ou Asn,
- Xaa^{j} représente la biphénylalanine (Bip), la phénylalanine ou la [bêta]-naphthylalanine,
- Xaa^{k} représente Thr ou Ala, et
- Xaa^{l} représente Gly, Val ou Leu,
- Xaa^{m} représente -NH₂ ou -OH,
les résidus d'acides aminés dans P1, P2 et P3 étant naturels ou non naturels, identiques ou différents, lesdits résidus de P1, P2 et P3 étant tous différents du résidu Lys, et P1, P2 et P3 ayant ou non une séquence commune,
caractérisé en ce que le procédé comprend la mise en présence du peptide de séquence générale (I) dérivé du récepteur CD4 avec un composé bifonctionnel portant deux groupes actifs, dont l'un au moins des deux groupes actifs est capable de former une liaison covalente avec la fonction amine libre (-NH₂) du résidu d'acide aminé Lys présent dans la séquence générale (I).

De préférence **P3** comporte au moins un acide aminé basique, tout préférentiellement ledit acide aminé basique est une arginine. La présence de résidus basiques dans cette portion du fragment du récepteur CD4 contribue à sa liaison avec la protéine gp120. Ainsi les inventeurs ont préféré introduire dans **P3** au moins un acide aminé basique, de préférence une arginine. Celle-ci maintient ainsi une charge basique qui n'est pas réactive pour une dérivatisation à pH7-8 mais qui s'est effectivement avéré utile pour la liaison du peptide miniCD4 avec la protéine gp 120.

Dans la présente demande, les expressions « peptide miniCD4 », « peptide CD4 » ou « miniCD4 », sont utilisées de manière indifférente pour désigner le peptide dérivé du récepteur CD4 comprenant ou étant constitué par la séquence générale (I) définie ci-dessus.

La présente invention requiert que le peptide dérivé du récepteur CD4 comporte dans sa séquence générale (I) un et un seul résidu d'acide aminé lysine (Lys), dans la position telle que définie dans la séquence générale (I).

Les résidus Cys dans la séquence générale (I) permet la formation de trois ponts disulfures nécessaires au repliement du miniCD4.

L'acide thiopropionique (TPA), lorsqu'il est en position N-terminale du peptide de séquence générale (I), permet de diminuer l'encombrement en N-ter et de s'affranchir de la présence d'une fonction amine.

Ainsi, selon un mode de réalisation préféré, Xaa^{f} représente TPA dans la séquence générale (I).

Dans la séquence générale (I), Xaa^{J} représente la Bip, Phe ou la [bêta]-naphthylalanine La biphénylalanine permet d'augmenter le contact avec la glycoprotéine gp120 au niveau de la cavité dans laquelle se loge la Phe 43 du récepteur CD4. Toutefois, un peptide miniCD4 selon l'invention avec une Phe pourrait être un meilleur mime de CD4 lorsqu'on analyse la structure du complexe miniCD4/gp120 (Huang CC et al., Structure. 2005 May;13(5):755-68).

Ainsi, selon un autre mode de réalisation préféré, Xaa^{J} représente Phe.

Le peptide de séquence générale (I) dérivé du récepteur CD4 présente une structure en hélice alpha suivie d'un feuillet bêta. Les acides aminés Xaa^{g}-Xaa^{h}-Xaaⁱ-Xaa^{j}-Cys-Xaa^{h}-Cys-Xaa^{l} participent majoritairement à la liaison sur gp120. Ces peptides présentent des IC₅₀ (affinité pour gp120) voisines de celles du sCD4 (CD4 soluble).

Le peptide de séquence générale (I) dérivé du récepteur CD4 peut être préparé par les techniques classiques de synthèse chimique en phase solide, par exemple selon la méthodologie de synthèse peptidique en phase solide Fmoc (« Fmoc solid Phase peptide synthesis, a practical approach », publié par W.C. Chan et P.D. White, Oxford university press, 2000), et/ou par recombinaison génétique.

De préférence, la séquence du peptide dérivé du récepteur CD4 de séquence générale (I) est choisie dans le groupe constitué par les séquences SEQ ID N°1 et SEQ ID N°2, avantageusement SEQ ID N°1.

On entend désigner par « composé bifonctionnel » dans la présente demande tout composé portant deux groupes actifs dont l'un au moins des deux groupes est capable de former une liaison covalente avec la fonction amine libre (-NH₂) du résidu d'acide aminé Lys de la séquence générale (I).

L'homme du métier connaît bien les composés bifonctionnels qui peuvent être utilisés dans le cadre de la présente invention. Notamment, le composé bifonctionnel selon la présente invention peut être choisi dans la liste suivante, non limitative : NHS-PEOₙ-Maleimide où n est compris entre 2 et 24, avantageusement n=2, 4, 8 ou 12, SMPT (4-succinimidyloxycarbonyl-méthyl-α-[2-pyridyldithio]toluène), Sulfo-LC-SMPT (4-sulfosuccinimidyl-6-méthyl-α-(2-pyridyldithio)toluamido]hexanoate)), Sulfo-KMUS (*N*-[k-maléïmidoundécanoyloxy]sulfosuccinimide ester), LC-SMCC (succinimidyl-4-[N-maléïmidométhyl]cyclohexane-1-carboxy-[6-amidocaproate]), KMUA (acide N-k-maléïmidoundécanoïque), Sulfo-LC-SPDP (sulfosuccinimidyl 6-(3'-[2-pyridyldithio]-propionamido)hexanoate), LC-SPDP (succinimidyl 6-(3-[2-pyridyldithio]-propionamido)hexanoate), SMPB (succinimidyl 4-[*p-*maléïmidophényl]butyrate), Sulfo-SMPB (sulfosuccinimidyl 4-[*p-*maléïmidophényl]butyrate), Sulfo-SIAB (*N-*sulfosuccinimidyl[4-iodoacétyl]aminobenzoate), SIAB (*N*-succinimidyl[4-iodoacétyl]aminobenzoate), Sulfo-EMCS ([N-e-maléïmidocaproyloxy]sulfosuccinimide ester), EMCA (acide N-e-maléïmidocaproïque), EMCS ([*N*-e-maléïmidocaproyloxy]succinimide ester), SMCC (succinimidyl 4-[*N-*maléïmidométhyl]cyclohexane-1-carboxylate), Sulfo-SMCC (sulfosuccinimidyl 4-[*N-*maléïmidométhyl]cyclohexane-1-carboxylate), MBS (m-maléïmidobenzoyl-N-hydroxy succinimide ester), Sulfo-MBS (m-maléïmidobenzoyl-N-hydroxysulfosuccinimide ester), GMBS (N-[g-maléïmidobutyryloxy]succinimide ester), Sulfo-GMBS (N-[g-maléïmidobutyryloxy] sulfosuccinimide ester), SPDP (N-succinimidyl-3-(2-pyridyldithio)propionate), SBAP (succinimidyl 3-[bromoacétamido]propionate), BMPS (N-[[bêta]-maléïmidopropyloxy]succinimide ester), BMPA (acide N-[bêta]-maléïmidopropionique), AMAS N-(a-maléïmidoacétoxy) succinimide ester), SIA (*N-*succinimidyl iodoacétate), SMPH (succinimidyl-6-[bêta-maléïmidopropionamido]hexanoate), SATA (N-succinimidyl-S-acétylthioacétate), SATP (N-succinimidyl-S-acétylthiopropionate).

Selon la présente invention, NHS-PEOₙ-Maleimide où n=2 est également appelé succinimidyl-[(N-maleimidopropionamido)-diethyleneglycol] ester, NHS-PEOₙ-Maleimide où n=4 est également appelé succinimidyl-[(N-maleimidopropionamido)-tetraethyleneglycol] ester,
NHS-PEOₙ-Maleimide où n=8 est également appelé succinimidyl-[(N-maleimidopropionamido)-octaethyleneglycol] ester,
NHS-PEOₙ-Maleimide où n=12 est également appelé succinimidyl-[(N-maleimidopropionamido)-dodecaethyleneglycol] ester.

Le groupe actif capable de former une liaison covalente avec la fonction amine libre (-NH₂) du résidu d'acide aminé Lys présent dans la séquence générale (I), peut être tout groupe ester actif.

De manière préférée, le groupe actif capable de former une liaison covalente avec la fonction amine libre (-NH₂) du résidu d'acide aminé Lys présent dans la séquence générale (I), est le groupe actif N-hydroxysuccinimide ester (NHS).

De manière encore préférée, les deux groupes actifs du composé bifonctionnel sont différents (groupe hétérobifonctionnel), et l'un des deux groupes représente le groupe actif NHS.

Selon un mode de réalisation particulièrement préféré, le composé bifonctionnel est le succinimidyl-6-[bêta-maléïmidopropionamido]hexanoate (SMPH).

La structure moléculaire du SMPH est la suivante :

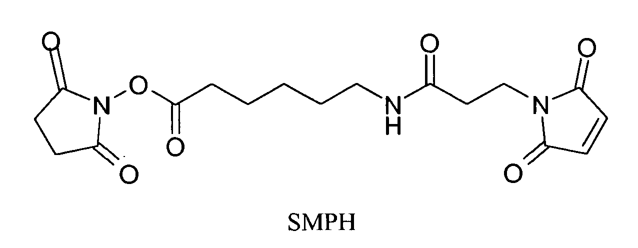

Selon un autre mode de réalisation particulièrement préféré, le composé bifonctionnel est choisi dans le groupe constitué par le N-succinimidyl-S-acétylthioacétate (SATA) et le N-succinimidyl-S-acétylthiopropionate (SATP).

La structure moléculaire du SATA est la suivante :

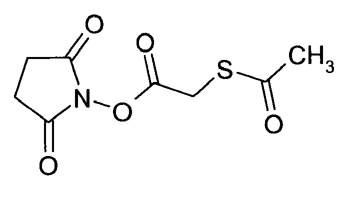

La structure moléculaire du SATP est la suivante :

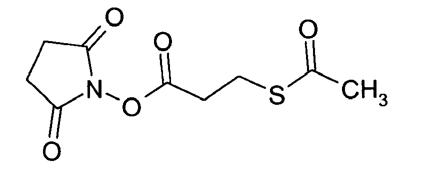

Selon encore un autre mode de réalisation préféré, le composé bifonctionnel est l'ester de succinimidyl-[(N-maléïmidopropionamido)-diéthylèneglycol], également appelé NHS-PEO₂-maléïmide l'ester de succinimidyl-[(N-maleimidopropionamido)-tetraethyleneglycol] également appelé NHS-PEO₄- maléïmide, l'ester de succinimidyl-[(N-maleimidopropionamido)-octaethyleneglycol] également appelé NHS-PEO₈-maléïmide, l'ester de succinimidyl-[(N-maleimidopropionamido)-dodecaethyleneglycol] également appelé NHS-PEO₁₂- maléïmide, de manière encore plus préférée le composé bifonctionnel est le NHS-PEO₂- maléïmide.

La structure moléculaire du NHS-PEO₂-maléïmide est la suivante :

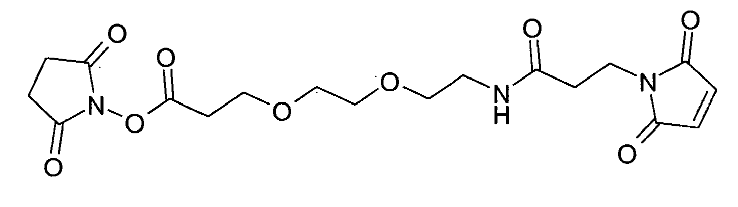

Les composés bifonctionnels peuvent être obtenus auprès de PIERCE (Rockford, IL).

De préférence encore, le procédé de préparation d'un peptide activé selon l'invention comprend une étape préliminaire de préparation du peptide dérivé du récepteur CD4 de séquence générale (I) dans lequel Xaa^{f} représente TPA, dans laquelle le peptide dérivé du récepteur CD4 de séquence générale (II) suivante :

P1 - Lys - Cys - P2 - Cys - P3 - Cys - Xaa^{g} - Xaa^{h} - Xaaⁱ - Xaa^{j} - Cys - Xaa^{k}-Cys-Xaa^{l}-Xaa^{m}, (II)

dans laquelle P1 à P3, Bip et Xaa^{g} à Xaa^{m} sont tels que définis dans la séquence générale (I) ci-dessus,
est mis en présence avec le N-succinimidyl 3-(2-pyridyldithio)propionate (SPDP) pour incorporer le TPA à l'extrémité N-terminale dudit peptide dérivé du récepteur CD4 de séquence générale (II).

La structure moléculaire du SPDP est la suivante :

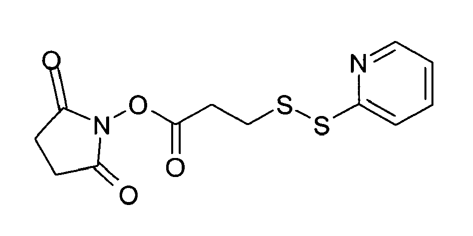

Selon un deuxième aspect, la présente invention a pour objet un peptide activé dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif capable de se coupler par liaison covalente à une molécule organique.

Comme exemples de groupes actifs capable de se coupler par liaison covalente à la molécule organique, on peut citer le groupe maléïmide, le groupe bromoacétyle, le groupe S-S-pyridinium. Lorsque le miniCD4 est activé par une fonction thiol protégée (ex : thioacétyle), il est possible de réaliser le couplage avec une molécule organique qui portera par exemple un groupe maléïmide. Cette possibilité peut être utilisée lorsque la fonctionnalisation du polysaccharide (ou polyanion) par une fonction thiol ou thioacétyle pose des problèmes. On parle alors de « couplage inverse ».

De préférence, le groupe actif est le groupe maléïmide.

La structure moléculaire du peptide activé selon la présente invention, dont le groupe actif est le groupe maléïmide, est la suivante lorsque SMPH est le composé bifonctionnel utilisé:

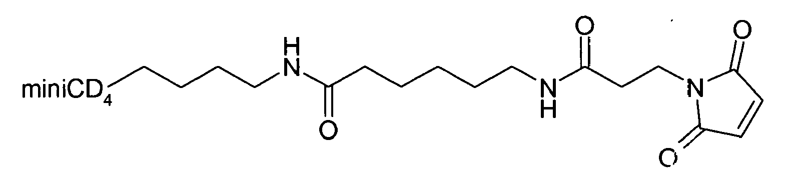

Dans la présente demande, l'expression « peptide miniCD4 activé SMPH » désigne un peptide activé selon l'invention, dont le résidu d'acide aminé Lys est lié de façon covalente, avantageusement par une liaison amine, au groupe actif maléïmide via un espaceur dérivé du SMPH.

Selon un autre mode de réalisation avantageux, la structure moléculaire du peptide activé selon la présente invention, dont le groupe actif est le groupe maléïmide, est la suivante lorsque NHS-PEO₂-maléïmid le composé bifonctionnel utilisé :

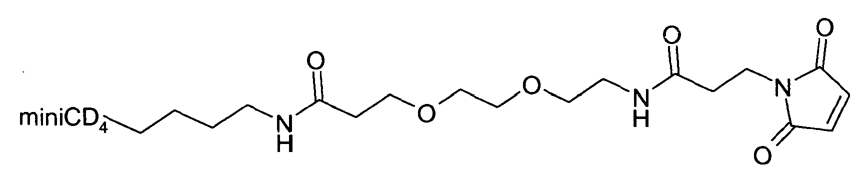

Dans la présente demande, l'expression « peptide miniCD4 activé maléïmide via un espaceur PEO₂ » désigne un peptide activé selon l'invention, dont le résidu d'acide aminé Lys est lié de façon covalente, avantageusement par une liaison amine, au groupe actif maléïmide via un espaceur PEO₂ .

Selon une autre préférence, le groupe actif est le groupe thioacétyle.

Par exemple, la structure moléculaire du peptide activé selon la présente invention, dont le groupe actif est le groupe thioacétyle, est la suivante lorsque SATA est le composé bifonctionnel utilisé :

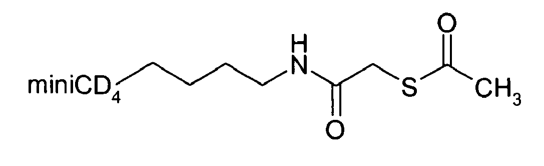

De même, la structure moléculaire du peptide activé selon la présente invention, dont le groupe actif est le groupe thioacétyle, est la suivante lorsque SATP est le composé bifonctionnel utilisé :

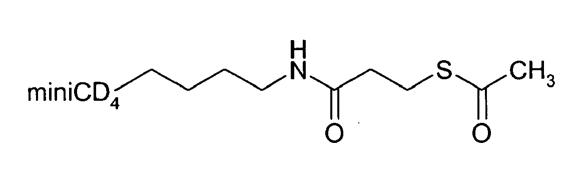

Le groupe thioacétyle est une forme protégée du groupe thiol. Pour déprotéger le groupe thiol, on utilise par exemple l'hydroxylamine. Cette étape est réalisée simultanément lors du couplage sur la fonction maléïmide portée par la molécule organique.

Dans la présente demande, les expressions « peptide miniCD4 activé SATA » et « peptide miniCD4 activé SATP » désignent un peptide activé selon l'invention, dont le résidu d'acide aminé Lys est lié de façon covalente, avantageusement par une liaison amine, à un groupe thiol protégé ( ex : thioacétyle) via un espaceur dérivé du SATA ou SATP.

Selon un troisième aspect, la présente invention a pour objet un procédé de préparation d'une molécule conjuguée comportant un peptide dérivé du récepteur CD4 couplé par liaison covalente à une molécule organique, ledit peptide dérivé du récepteur CD4 comprenant ou étant constitué par la séquence générale (I) telle que définie ci-dessus, caractérisé en ce que le procédé comprend la mise en présence du peptide activé selon l'invention tel que défini ci-dessus avec la molécule organique. Le peptide activé dérivé du récepteur CD4 comprend la séquence générale (I) telle que définie ci-dessus, dans lequel le résidu d'acide aminé Lys est lié de façon covalente, avantageusement par une liaison amine, à un groupe actif capable de se coupler par liaison covalente à une molécule organique.

Selon un mode réalisation particulier, le groupe actif est le groupe maléïmide et la molécule organique porte une fonction thiol ou thioacétyle.

De préférence, la molécule organique portant une fonction thiol est le peptide GPR1 de séquence choisie dans le groupe constitué par les séquences SEQ ID N°3 et SEQ ID N°4, avantageusement SEQ ID N°3.

Le peptide GPR1 de séquence SEQ ID N°3 ou SEQ ID N°4 est un peptide synthétique dérivé de la région extracellulaire N-terminale du récepteur couplé à la protéine G, GPR1 (Jinno-Oue et al., J Biol Chem. 2005 Sep 2;280(35):30924-34. Epub 2005 May 26).

Selon une autre préférence, la molécule organique portant une fonction thiol est choisie dans le groupe constitué par les peptides comportant essentiellement des résidus acides (acide aspartique, acide glutamique,...), les peptides comportant essentiellement des résidus phosphorylables (Ser, Thr, Tyr,...), et les peptides comportant essentiellement des résidus sulfatables (Ser, Thr, Tyr,...).

Selon une autre préférence, la molécule organique portant une fonction thiol est un polyanion modifié portant la fonction thiol ou thioacétyle.

Selon l'invention, le polyanion, ou polysaccharide, peut être choisi avantageusement dans le groupe constitué de l'héparine, de l'héparane sulfate, et d'un polyanion équivalent à l'héparine ou à l'héparane sulfate. Il s'agit par exemple du dextran sulfate (marque de commerce, UENO FINE CHEMICALS), du curdlan sulfate (marque de commerce, AJINOMOTO), du naphtalène-2 sulfonate polymère (marque de commerce, PROCEPT), du pentosan polysulfate (marque de commerce, BAKER NORTON PHARM ; HOECHST), ou du resobene (marque de commerce).

Il est préférable que le polyanion ne soit pas trop long, car il aurait une activité anticoagulante, non souhaitée dans la présente invention, et formerait des liaisons aspécifiques avec différentes protéines, notamment la thrombine ou l'antithrombine III. Sa longueur sera de préférence similaire à une chaîne d'héparine ayant un degré de polymérisation tel que défini ci-dessous. Le polyanion présente de préférence au moins deux groupes anioniques par disaccharide.

Selon la présente invention, lorsque le polyanion est de l'héparine ou de l'héparane sulfate, il aura de préférence un degré de polymérisation dp de 10 à 24, avantageusement de 12 à 24, de préférence de 16 à 22. Selon l'invention, l'héparine, l'héparane sulfate ou le polyanion équivalent à l'héparine ou l'héparane sulfate peut avoir un degré de polymérisation dp de 12 à 20, par exemple de 15 à 17.

On peut citer par exemple le dodécasaccharide d'héparine (HP₁₂).

Selon un mode de réalisation particulier, le polyanion modifié portant la fonction thiol ou thioacétyle est choisi dans le groupe constitué par l'héparine et l'héparane sulfate et a un degré de polymérisation dp de 10 à 24.

Selon un autre mode de réalisation particulier, le groupe actif est le groupe thioacétyle et la molécule organique porte une fonction maléïmide ou halogène.

Selon l'invention, le polyanion peut être préparé par dépolymérisation partielle d'héparine ou d'héparane sulfate par une méthode enzymatique, par exemple au moyen d'héparinase, ou chimique, par exemple au moyen d'acide nitreux. Lorsqu'ils sont obtenus chimiquement, les héparanes peuvent être définis par la présence de glucosamine N-sulfatée ou N-acétylée, ou non substituée en position N, liée à un acide uronique (acide glucuronique ou acide iduronique) avec une proportion variable de groupe sulfate. Des mimes structuraux de ces oligosaccharides peuvent être obtenus par synthèse chimique.

Les avantages d'une telle approche synthétique par rapport à une conjugaison de composés recombinants ou issus de sources naturelles sont multiples. Dans l'optique d'une utilisation thérapeutique, les composés de synthèse sont toujours préférés, car outre une structure parfaitement définie, on évite toute contamination par des agents pathogènes et en particulier des protéines prions dans le cas de fragments d'HP. De plus, les fragments synthétiques d'HP sont nettement plus homogènes que leurs équivalents naturels. Ainsi par exemple, l'HP₁₂ synthétique est totalement dépourvu des groupements 3-*O*-sulfates qui sont à l'origine de l'activité antithrombotique de l'héparine.

Les conditions opératoires des procédés de préparation selon l'invention du peptide activé et de la molécule conjuguée, sont bien connus de l'homme de l'art et pourront être adaptées si besoin est.

Selon un quatrième aspect, la présente invention a pour objet une molécule conjuguée comportant un peptide dérivé du récepteur CD4 comprenant ou étant constitué par la séquence générale (I) telle que définie ci-dessus couplé à une molécule organique, dans laquelle le peptide dérivé du récepteur CD4 et la molécule organique sont couplés l'un à l'autre par un bras espaceur, et dans laquelle le résidu d'acide aminé Lys de la séquence générale (I) forme une liaison amine avec le bras espaceur.

La présente invention a également pour objet une molécule conjuguée comportant un peptide dérivé du récepteur CD4 comprenant ou étant constitué par la séquence générale (I) telle que définie ci-dessus couplé à une molécule organique, ladite molécule conjuguée étant susceptible d'être obtenue par le procédé selon l'invention de préparation d'une molécule conjuguée.

Dans la présente demande, on entend désigner par bras espaceur tout agent de liaison entre le peptide miniCD4 selon l'invention et la molécule organique, ledit bras espaceur variant selon le composé bifonctionnel utilisé.

Il s'agit de préférence d'une molécule conjuguée selon l'invention, dans laquelle la séquence du peptide dérivé du récepteur CD4 comprend ou est constituée par la séquence générale (I), de préférence la séquence SEQ ID N°1, et la molécule organique portant une fonction thiol est le peptide GPR1 de séquence choisie dans le groupe constitué par les séquences SEQ ID N°3 et SEQ ID N°4, avantageusement SEQ ID N°3.

Le couplage selon l'invention du peptide GPR1 au peptide dérivé du récepteur CD4 de séquence générale (I) est capable de se fixer sur la glycoprotéine gp120 du VIH et de bloquer de façon concomitante l'attachement du virus à CD4 et aux corécepteurs CXCR4 et CCR5.

La structure moléculaire d'une telle molécule conjuguée, comportant un peptide dérivé du récepteur CD4 de séquence générale (I) couplé au peptide GPR1 de séquence SEQ ID N°3 ou SEQ ID N°4, est la suivante lorsque le SMPH a été utilisé pour le couplage :

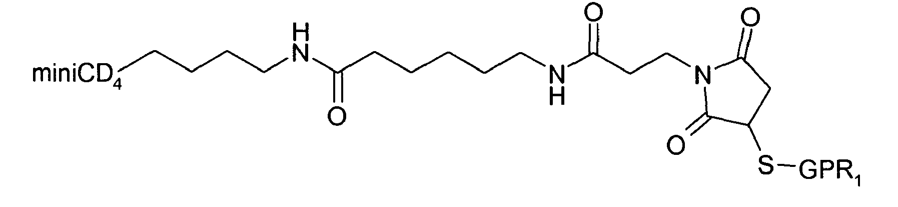

Selon une autre préférence, il s'agit d'une molécule conjuguée selon l'invention, dans laquelle la séquence du peptide dérivé du récepteur CD4 comprend ou est constituée par la séquence générale (I), de préférence la séquence SEQ ID N°1, et la molécule organique portant une fonction thiol est le polyanion modifié portant la fonction thiol ou thioacétyle.

La structure moléculaire d'une telle molécule conjuguée, comportant un peptide dérivé du récepteur CD4 de séquence générale (I) couplé au polyanion modifié portant la fonction thiol ou thioacétyle, est la suivante lorsque le SMPH a été utilisé pour le couplage :

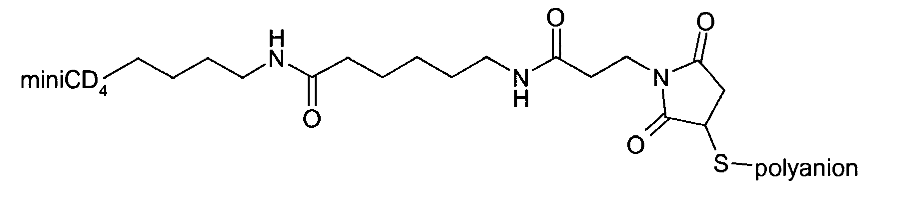

Il peut également s'agir de la molécule conjuguée suivante, lorsque le NHS-PEO₂-maléïmide est le composé bifonctionnel utilisé :

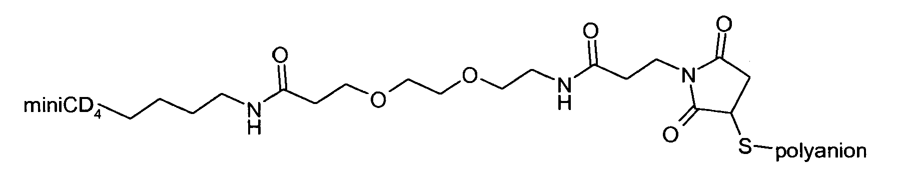

De préférence, le polyanion modifié portant la fonction thiol ou thioacétyle est choisi dans le groupe constitué par l'héparine et l'héparane sulfate et a un degré de polymérisation dp de 10 à 24.

Selon un autre mode de réalisation, la molécule conjuguée selon l'invention comprend le peptide dérivé du récepteur CD4 comprenant ou étant constitué par la séquence générale (I), de préférence la séquence SEQ ID N°1, et une molécule organique portant une fonction maléïmide ou halogène.

Par exemple, la structure moléculaire d'une telle molécule conjuguée, comportant un peptide dérivé du récepteur CD4 de séquence générale (I) couplé à une molécule organique portant une fonction maléïmide, est la suivante lorsque le SATA a été utilisé pour le couplage :

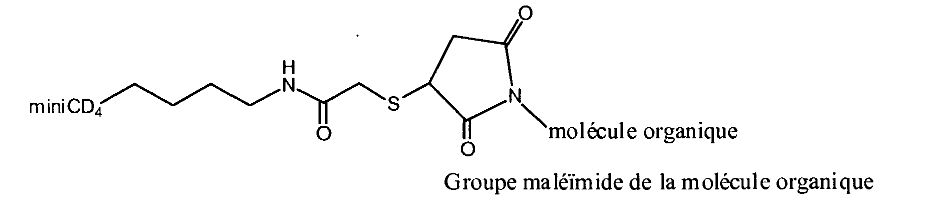

Il résulte de la présente invention que la molécule conjuguée telle que définie ci-dessus comporte un bras espaceur, dont la longueur varie selon le composé bifonctionnel utilisé.

Selon encore un autre mode de réalisation, la molécule conjuguée selon l'invention comprend le peptide dérivé du récepteur CD4 comprenant ou étant constitué par la séquence générale (I), de préférence la séquence SEQ ID N°1, et la molécule organique portant une fonction thiol choisie dans le groupe constitué par les peptides comportant essentiellement des résidus acides (acide aspartique, acide glutamique,...), les peptides comportant essentiellement des résidus phosphorylables (Ser, Thr, Tyr,...), et les peptides comportant essentiellement des résidus sulfatables (Ser, Thr, Tyr,...).

La présente invention a encore pour objet l'utilisation du peptide activé dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif maléïmide, pour le couplage par liaison covalente à une molécule organique comportant une fonction thiol ou thioacétyle.

Un autre objet de la présente invention est l'utilisation du peptide dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif thiol protégé (ex : thioacétyle), pour le couplage par liaison covalente à une molécule organique comportant une fonction maléïmide ou halogène.

L'invention a également pour objet l'utilisation du peptide activé dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif maléïmide permettant le couplage par liaison covalente à une molécule organique comportant une fonction thiol ou thioacétyle, pour la fabrication d'un médicament destiné à un traitement antiviral.

L'invention a encore pour objet l'utilisation du peptide activé dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif thiol protégé (ex : -thioacétyle) permettant le couplage par liaison covalente à une molécule organique comportant une fonction maléïmide ou halogène, pour la fabrication d'un médicament destiné à un traitement antiviral.

De préférence, l'invention a pour objet l'utilisation du peptide activé dérivé du récepteur CD4 comprenant ou étant constitué par la séquence générale (I) telle que définie ci-dessus, dans lequel le résidu d'acide aminé Lys est lié de façon covalente, avantageusement par une liaison amine, à un groupe actif maléïmide permettant le couplage par liaison covalente à une molécule organique comportant une fonction thiol ou thioacétyle, pour la fabrication d'un médicament destiné à traiter le sida.

L'invention a encore pour objet une molécule conjuguée selon la présente invention, pour son utilisation en tant que médicament.

Selon encore un autre aspect, l'invention a pour objet l'utilisation de la molécule conjuguée selon la présente invention pour la fabrication d'un médicament destiné au traitement du sida.

L'invention concerne également une méthode de traitement antiviral, de préférence une méthode de traitement contre le sida, comprenant l'utilisation d'une molécule conjuguée selon la présente invention.

De préférence, la molécule conjuguée selon la présente invention est une molécule conjuguée dans laquelle la séquence générale (I) est la séquence SEQ ID N°1, et la molécule organique portant une fonction thiol est le peptide GPR1 de séquence choisie dans le groupe constitué par les séquences SEQ ID N°3 et SEQ ID N°4, avantageusement SEQ ID N°3.

Selon une autre préférence, la molécule conjuguée selon la présente invention est une molécule conjuguée dans laquelle la séquence générale (I) est la séquence SEQ ID N°1, et la molécule organique portant une fonction thiol choisie dans le groupe constitué par les peptides comportant essentiellement des résidus acides (acide aspartique, acide glutamique,...), les peptides comportant essentiellement des résidus phosphorylables (Ser, Thr, Tyr,...), et les peptides comportant essentiellement des résidus sulfatables (Ser, Thr, Tyr,...).

Selon une autre préférence, la molécule conjuguée selon la présente invention est une molécule conjuguée dans laquelle la séquence générale (I) est la séquence SEQ ID N°1, et la molécule organique portant une fonction thiol est le polyanion modifié portant la fonction thiol ou thioacétyle.

Les modes de réalisation décrits précédemment s'appliquent à ces différents aspects de l'invention.

Les exemples et figures qui suivent permettent d'illustrer la présente invention, mais n'en limitent pas la portée.

### FIGURES

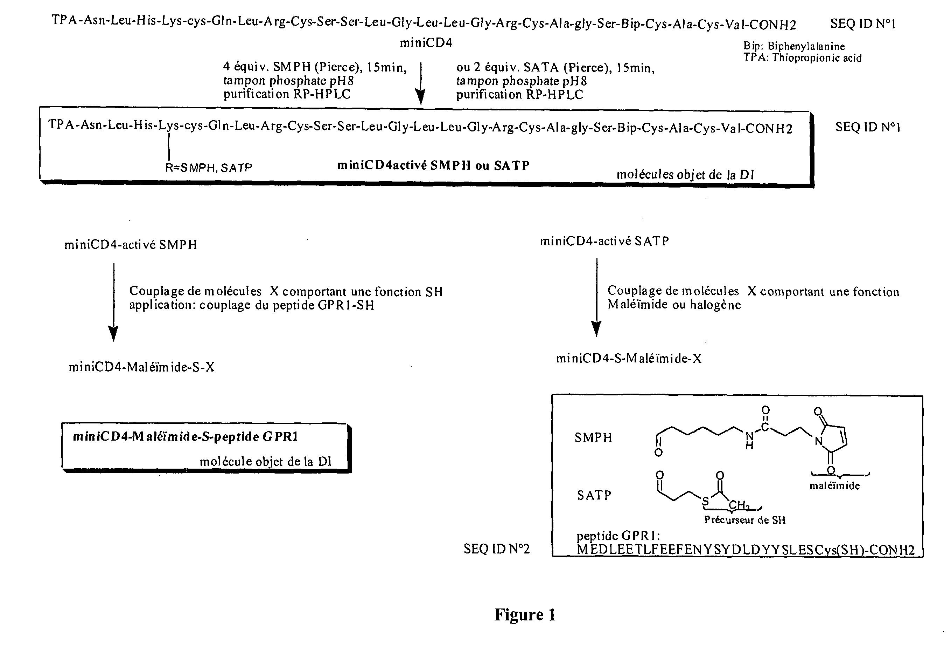

### Figure 1 : Schéma de synthèse du peptide activé SMPH ou SATA/SATP dérivé du récepteur CD4, et de la molécule conjuguée miniCD4-GPR1.

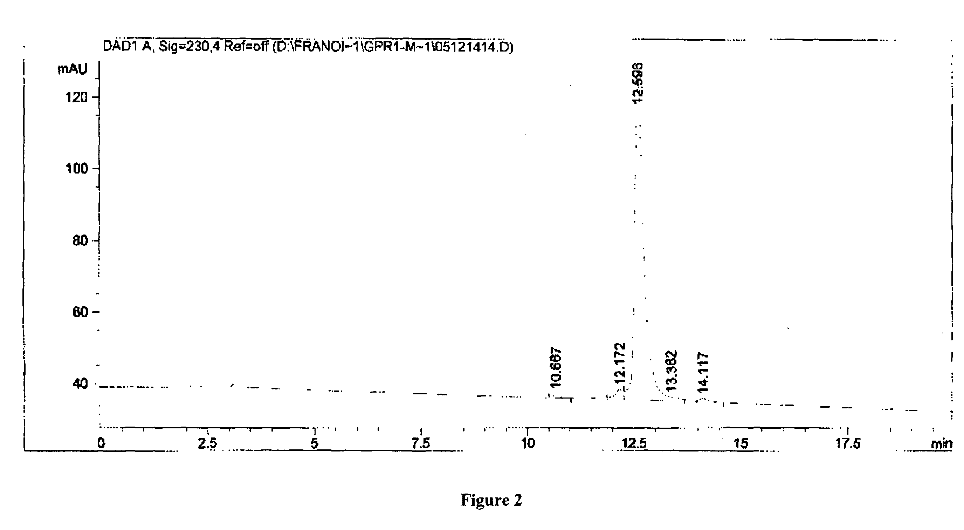

### Figure 2 : Elugramme HPLC final de la molécule conjuguée miniCD4-GPR1.

Epsilon ds 50µl TFA/CH3CN 35 % ; inj 10 µl ds 35-55
Nom de l'échantillon : GPR1-mCD4

### Rapport pourcentage surface :

| | |
|---|---|
| Trié par | : Signal |
| Multiplicateur | : 1.0000 |
| Dilution | : 1.0000 |

Utilise multiplicateur et facteur de dilution avec standard interne
Signal 1 : DAD1 A, Sig=230,4 Ref=off

**Tableau 1**

| **Pic n°** | **Temps de Rétention [min]** | **Type** | **Largeur [min]** | **Surface [mAU*s]** | **Hauteur [mAU]** | **Surface %** |
|---|---|---|---|---|---|---|
| 1 | 10.667 | BB | 0.1595 | 14.20322 | 1.29982 | 0.9401 |
| 2 | 12.172 | VV | 0.1905 | 39.30429 | 2.93002 | 2.6016 |
| 3 | 12.596 | VV | 0.2345 | 1422.81519 | 89.94869 | 94.1780 |
| 4 | 13.382 | VV B | 0.2054 | 18.64892 | 1.12492 | 1.2344 |
| 5 | 14.117 | BV | 0.2103 | 15.80048 | 1.02216 | 1.0459 |
| Totaux | | | | 1510.77210 | 96.32562 | |

| | | | | | | |
|---|---|---|---|---|---|---|
| Résultats obtenus avec intégrateur amélioré | | | | | | |

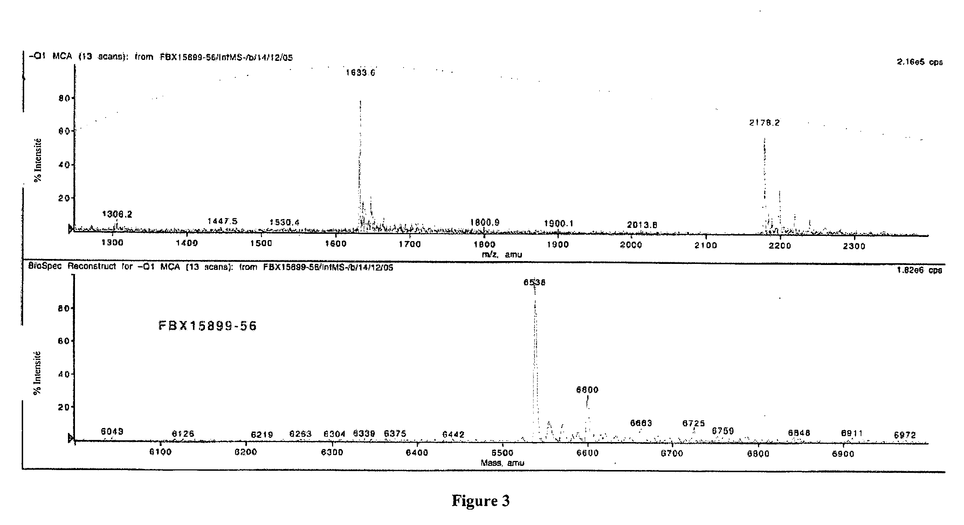

### Figure 3 : Spectre de masse de la molécule conjuguée miniCD4-GPR1.

Copie 3 du calcul de l'hypermasse pour -Q1 MCA (13 balayages) : à partir de FBX15899-56/InfMS-/b/14/12/05
Critères utilisés pour le calcul de l'hypermasse
Agent : , Masse : 1.0079, Charge : 1, Agent perdu
Tolérance pour l'estimation de la charge : 0.1000
Tolérances entre les estimations de masse : 20.0000

**Tableau 2**

| **Pic** | **Intensité** | **Charge** | **Charge calculée** | **Estimation de l'hypermasse** |
|---|---|---|---|---|
| 1306.33 | 10500.00 | 5 | 4.99783 | 6536.67 |
| 1633.34 | 215500.00 | 4 | 3.99783 | 6537.38 |
| 2178.24 | 139000.00 | 3 | 2.99935 | 6537.74 |

| | | | | |
|---|---|---|---|---|
| Masse finale estimée : 6537.26 Ecart-type : 0.54 | | | | |

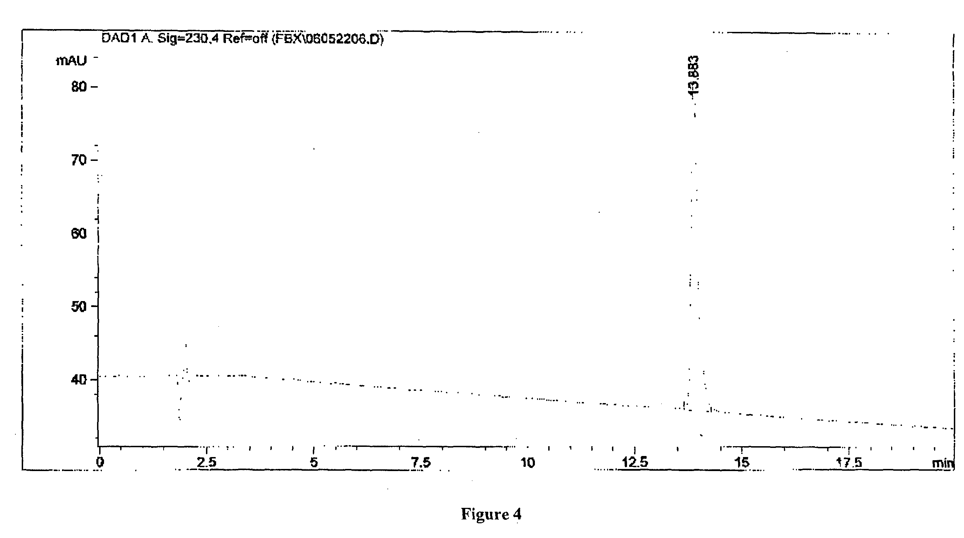

### Figure 4 : Elugramme HPLC final du peptide miniCD4 activé SATP

| | |
|---|---|
| 25-45 en 20min | |
| Nom de l'échantillon | : FBX13082-186-2 |
| Rapport pourcentage surface : | |
| Trié par | : Signal |
| Multiplicateur | : 1.0000 |
| Dilution | : 1.0000 |

Utilise multiplicateur et facteur de dilution avec standard interne
Signal 1 : DAD1 A, Sig=230,4 Ref=off

**Tableau 3**

| **Pic n°** | **Temps de Rétention [min]** | **Type** | **Largeur [min]** | **Surface [mAU*s]** | **Hauteur [mAU]** | **Surface %** |
|---|---|---|---|---|---|---|
| 1 | 13.883 | VV | 0.1668 | 520.71381 | 46.44330 | 100.0000 |
| Totaux | | | | 520.71381 | 46.44330 | |

| | | | | | | |
|---|---|---|---|---|---|---|
| Résultats obtenus avec intégrateur amélioré | | | | | | |

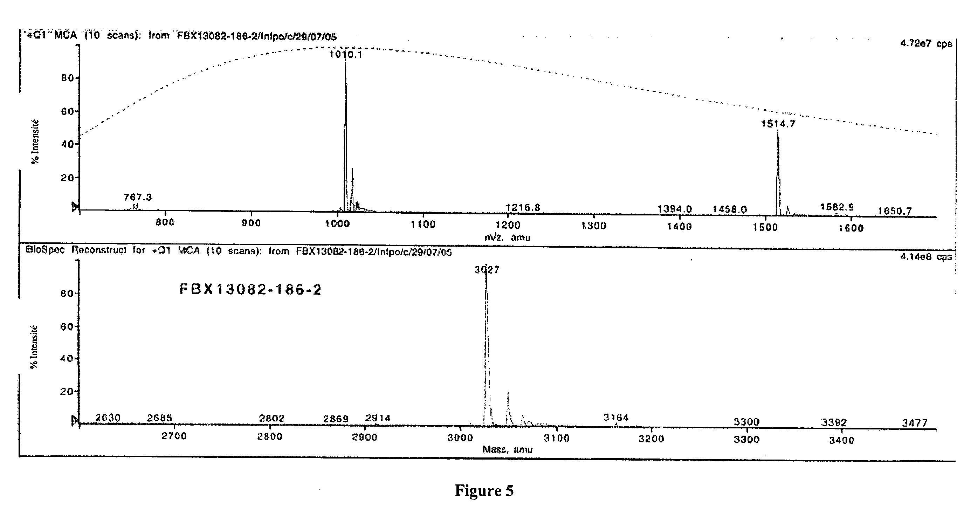

### Figure 5 : Spectre de masse du peptide miniCD4 activé SATP

Copie du calcul de l'hypermasse pour +Q1 MCA (10 balayages): à partir de FBX13082-186-2/Infpo/c/29/07/05
Critères utilisés pour le calcul de l'hypermasse
Agent : , Masse : 1.0079, Charge : 1, Agent gagné
Tolérance pour l'estimation de la charge : 0.1000
Tolérances entre les estimations de masse : 20.0000

**Tableau 4**

| **Pic** | **Intensité** | **Charge** | **Charge calculée** | **Estimation de l'hypermasse** |
|---|---|---|---|---|
| 757.72 | 125000.00 | 4 | 3.99687 | 3026.85 |
| 1010.22 | 45016000.00 | 3 | 2.99687 | 3027.64 |
| 1514.73 | 23987000.00 | 2 | 2.00039 | 3027.45 |

| | | | | |
|---|---|---|---|---|
| Masse finale estimée : 3027.31 Ecart-type : 0.41 | | | | |

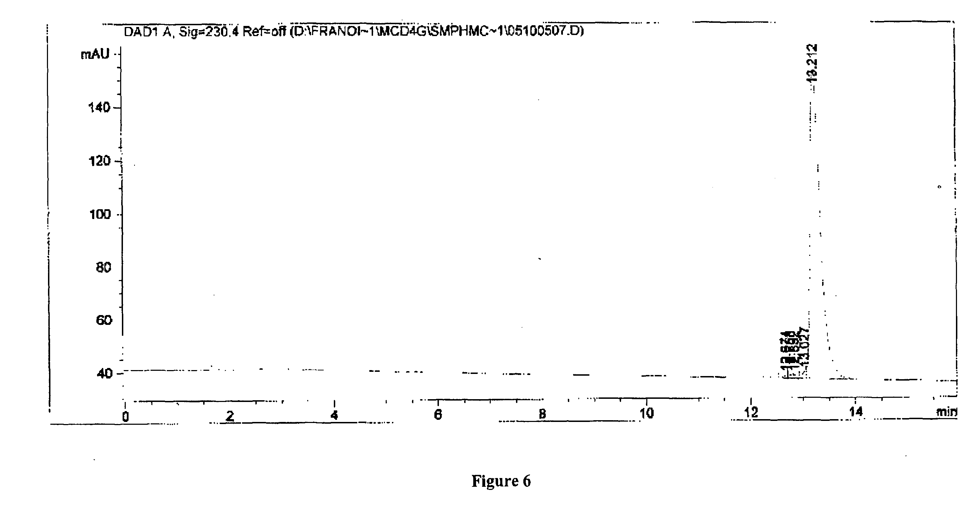

### Figure 6 : Elugramme HPLC final du peptide miniCD4 activé SMPH.

Environ 2 mg/ml
inj 5 µl ds 25-45
Nom de l'échantillon : FBX13082-190

### Rapport pourcentage surface :

| | |
|---|---|
| Trié par | : Signal |
| Multiplicateur | : 1.0000 |
| Dilution | : 1.0000 |

Utilise multiplicateur et facteur de dilution avec standard interne
Signal 1 : DAD1 A, Sig=230,4 Ref=off

**Tableau 5**

| **Pic n°** | **Temps de Rétention [min]** | **Type** | **Largeur [min]** | **Surface [mAU*s]** | **Hauteur [mAU]** | **Surface %** |
|---|---|---|---|---|---|---|
| 1 | 12.674 | BV F | 0.0909 | 6.52587 | 1.07449 | 0.3925 |
| 2 | 12.753 | VV | 0.0714 | 6.31252 | 1.19955 | 0.3797 |
| 3 | 12.832 | VV | 0.0909 | 8.14971 | 1.23625 | 0.4902 |
| 4 | 13.027 | VV F | 0.0859 | 16.22212 | 2.63102 | 0.9758 |
| 5 | 13.212 | VB | 0.2055 | 1625.25330 | 120.40638 | 97.7617 |
| Totaux | | | | 1662.46352 | 126.54769 | |

| | | | | | | |
|---|---|---|---|---|---|---|
| Résultats obtenus avec intégrateur amélioré | | | | | | |

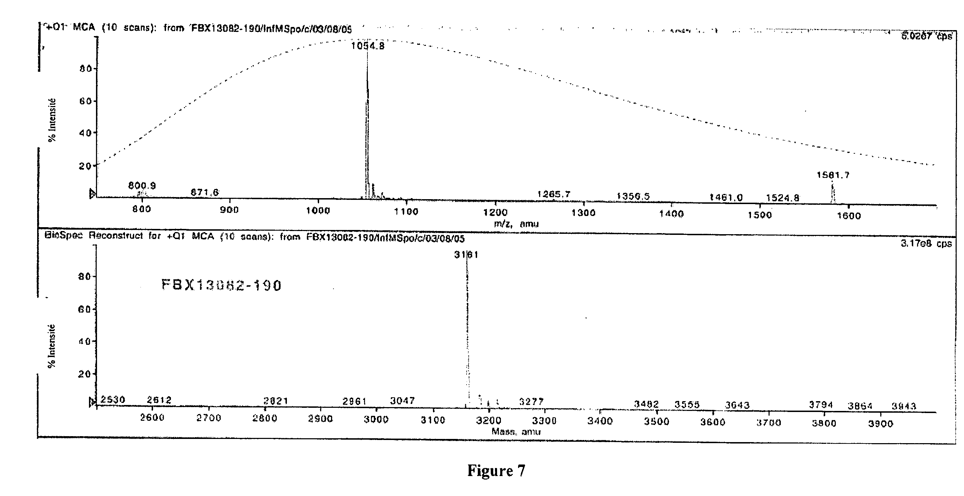

### Figure 7 : Spectre de masse du peptide miniCD4 activé SMPH

Copie du calcul de l'hypermasse pour +Q1 MCA (10 balayages): à partir de FBX13082-190/InfMSpo/c/03/08/05
Critères utilisés pour le calcul de l'hypermasse
Agent : , Masse : 1.0079, Charge : 1, Agent gagné
Tolérance pour l'estimation de la charge : 0.1000
Tolérances entre les estimations de masse : 20.0000

**Tableau 6**

| Pic | Intensité | Charge | Charge calculée | Estimation de l'hypermasse |
|---|---|---|---|---|
| 791.29 | 322500.00 | 4 | 4.00218 | 3161.12 |
| 1054.52 | 41316500.00 | 3 | 3.00218 | 3160.54 |
| 1581.43 | 7411500.00 | 2 | 1.99944 | 3160.84 |

| | | | | |
|---|---|---|---|---|
| Masse finale estimée : 3160.83 Ecart-type : 0.29 | | | | |

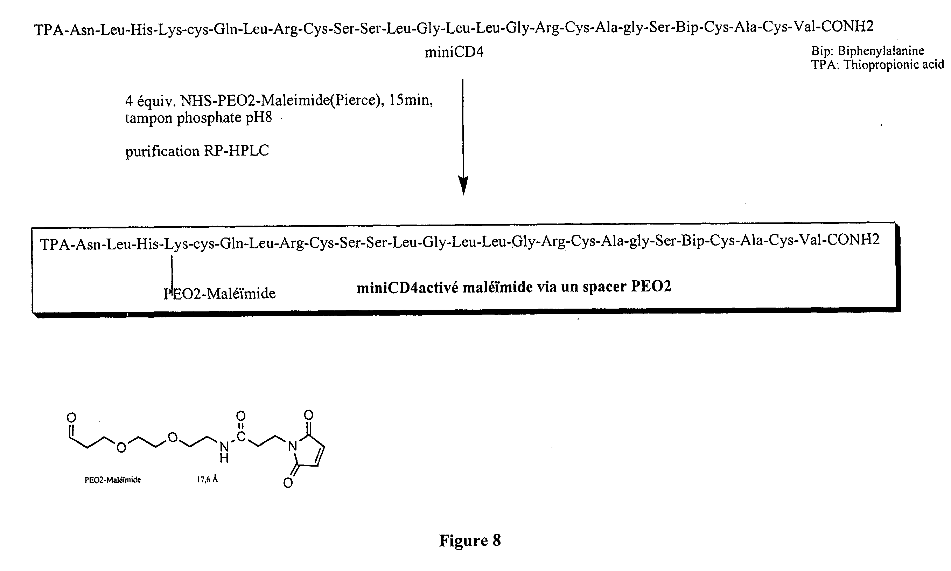

### Figure 8 : Schéma de synthèse du peptide miniCD4 activé maléïmide via un espaceur PEO₂.

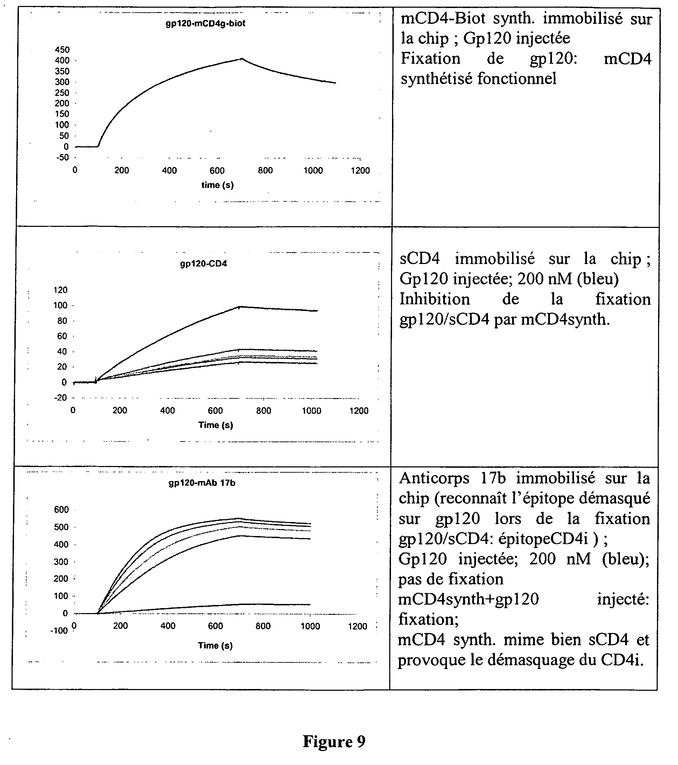

### Figure 9 : Interaction miniCD4 et gp120

L'affinité des miniCD4 synthétisés pour la gp120 a été évaluée par Biacore. Les résultats confirment que le mini CD4 que les Inventeurs ont « designé » comportant une seule lysine est un mime fonctionnel de la protéine CD4.

### Figure 10

Chromatographie HPLC du dérivé maleïmide, mini-CD4-PEO₂ Prouvant que ce dernier est obtenu avec une pureté finale de 77%, comme indiqué dans l'exemple V de la description.

### Figure 11

Spectre de masse, montrant la masse de dérivé obtenu (3205,3938)

### EXEMPLES

### Exemple I Schémas de synthèse

### I.1 Schéma de synthèse de la méthode de couplage entre un peptide miniCD4 et un polyanion à laquelle il est fait allusion dans la demande WO 03/089000 (Najjam S. et al.,Cytokine 1997, 9 (12) : 1013-1022).

### Couplage d'une fonction amine sur l'extrémité réductrice d'un sucre

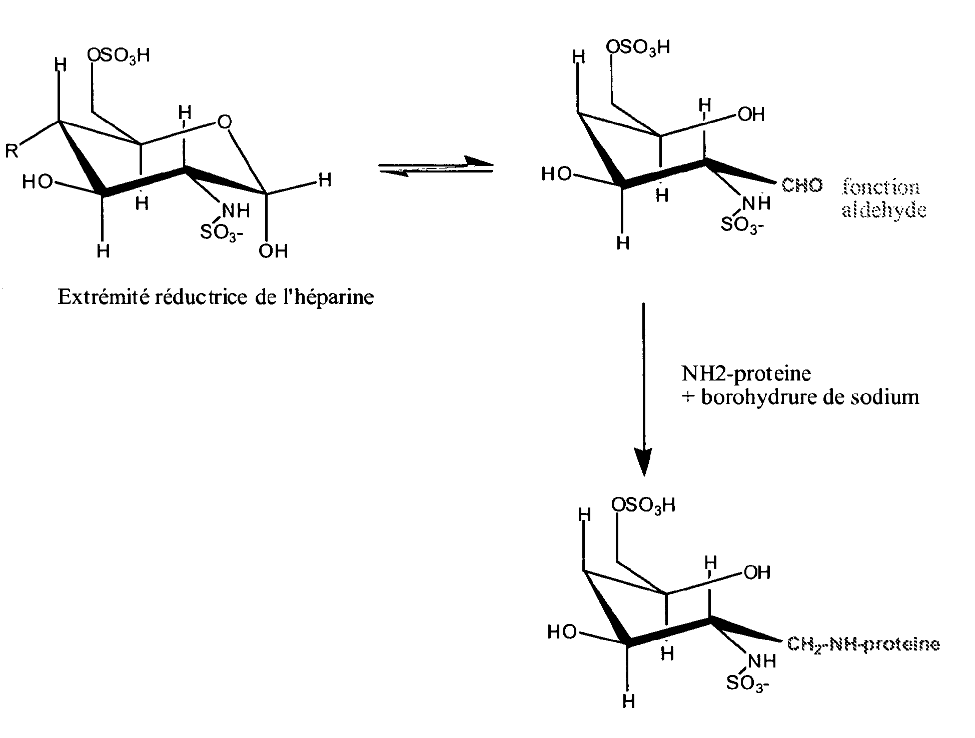

### I.2 Schéma de synthèse d'une méthode de couplage selon la présente invention

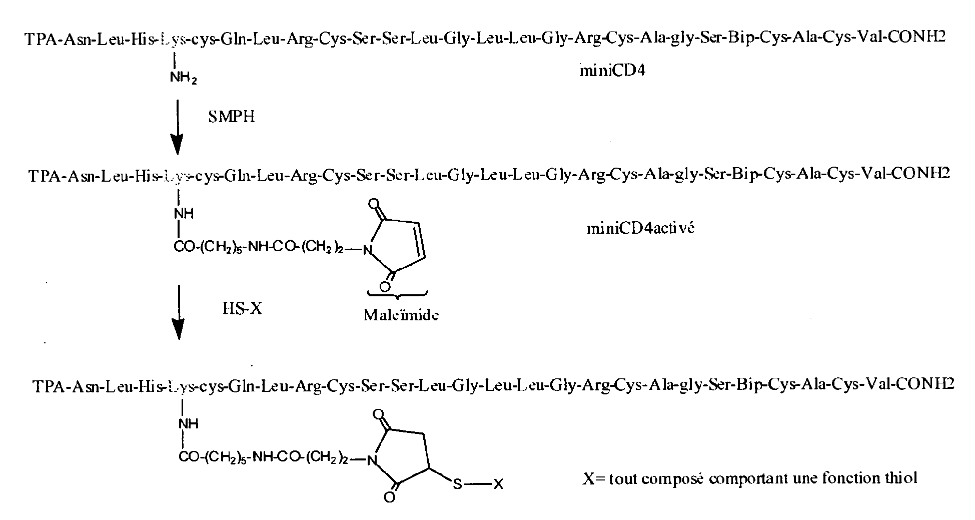

Le mode d'activation du miniCD4 via l'incorporation de la fonction maléïmide permet le couplage de tout composé comportant une fonction thiol libre (SH) ou une fonction thiol masquée (thioacétyle par exemple). Ce miniCD4 activé permet entre autre, l'obtention de conjugués covalents miniCD4-héparine, dans la mesure où l'héparine (ou tout autre polysaccharide) aura été préalablement dérivatisé par une fonction thiol.

### Exemple II Synthèse chimique de mini-CD4 activés avec du SMPH ou du SATP

### II.1 Synthèse de mini-CD4

Un mini-peptide CD4 a été synthétisé conformément à la méthodologie de synthèse peptidique en phase solide Fmoc (« Fmoc solid Phase peptide synthesis, a practical approach », publié par W.C. Chan et P.D. White, Oxford university press, 2000) sur un synthétiseur de peptides Applied Biosystems 433. En partant de 0,1 mmole de résine amide-Fmoc, l'élongation par étapes de la chaîne peptidique a été réalisée par couplage de 10 équivalents d'acides aminés protégés par Fmoc et activés avec un mélange HATU/DIEA. La fonction thiopropionyle N-terminale a été introduite par couplage de SPDP (1,6 équivalent dans du DMF) sur le peptide-résine.

Après clivage par du TFA/H₂O/EDT/TIS (94/2,5/2,5/1), le peptide a été récupéré par précipitation dans de l'éther diéthylique froid. Après lyophilisation, le peptide brut (156 mg) a été réduit pendant une nuit par du DTT dans une solution d'acide acétique à 20 % et purifié par MPLC en phase inverse sur une colonne Nucleoprep C1 18, 20 µm, 100 Å (26 X 313 mm) en utilisant un gradient linéaire de 30 à 90 % de B dans A, sur une période de 60 min, à un débit de 20 ml/min (B= 80 % de CH₃CN/ 20 % de TFA aqueux à 0,08 %; A= 100 % de TFA aqueux à 0,08 %). Les fractions pures ont été rassemblées et lyophilisées. Le peptide a ensuite été replié par traitement au GSH/GSSG pendant une nuit. Le peptide replié a été purifié par MPLC en utilisant un gradient de 20 à 80 % sur une période de 60 min, donnant lieu à 8,7 mg de mini-CD4. La pureté (93,5 %) a été vérifiée par HPLC analytique en phase inverse sur une colonne Nucleosil C18, 5 µm, 300 Å (4,6 X 150 mm) en utilisant un gradient linéaire de 25 à 35 % de CH₃CN dans du TFA aqueux à 0,08 %, sur une période de 20 min, à un débit de 1 ml/min (temps de rétention = 15,44 min).
ES⁺MS : 2896,32 ± 0,23; attendu : 2896,49 ; Rendement : 5,5 %.

### II.2 Mini-CD4 activé avec du SMPH

Le groupement maléïmide a été introduit sur la chaîne latérale de Lys du mini-CD4 par réaction de 4 équivalents de SMPH dans un tampon phosphate pH = 8. La réaction a été contrôlée par HPLC. Après 15 min, on a atteint 100 % du couplage. Après purification sur une colonne Nucleosil C18 semi-préparative pour HPLC en phase inverse, 5 µm, 300 Å (10 X 250 mm), en utilisant un gradient linéaire de 25 à 45 % de CH₃CN dans du TFA aqueux à 0,08 %, sur une période de 20 min, à un débit de 6 ml/min, la pureté finale (97,7 %) du mini-CD4 activé avec le SMPH a été contrôlée par RP-HPLC analytique en utilisant un gradient linéaire de 25 à 45 % (temps de rétention = 13,21 min).
ES⁺MS : 3160,83 ± 0,29; attendu : 3160,78. Rendement : 1,9 mg (67 %).

### II.3 Mini-CD4 activé avec du SATP

Le groupe thioacétyle a été introduit sur la chaîne latérale de Lys du mini-CD4 par réaction de 1 équivalent de SATP dans un tampon phosphate pH = 8. La réaction a été contrôlée par HPLC. Après 3 min, on a atteint 46% du couplage. Le candidat mini-CD4 activé avec le SATP a été isolé sur une colonne Nucleosil C18 semi-préparative pour HPLC en phase inverse, 5 µm, 300 Å (10 X 250 mm), en utilisant un gradient linéaire de 20 à 40 % de CH₃CN dans du TFA aqueux à 0,08 %, sur une période de 20 min, à un débit de 6 ml/min. La pureté finale (100 %) du mini-CD4 activé avec le SATP a été contrôlée par RP-HPLC analytique en utilisant un gradient linéaire de 25 à 45 % (temps de rétention = 13,88 min).
ES⁺MS: 3027,31 ± 0,41; attendu : 3027,66. Cette réaction de couplage a été réalisée une fois et pourrait être optimisée en ajoutant directement 2 équivalents de SATP.

### Exemple III Synthèse chimique d'un peptide mini-CD4-GPR1

### III.1 Synthèse de GPR1

Un peptide GPR1 a été synthétisé en utilisant la synthèse peptidique en phase solide Fmoc conventionnelle, comme décrit pour le mini-CD4. Un résidu Cys a été incorporé à l'extrémité C-terminale de la séquence de GPR1, pour permettre le couplage spécifique à la fonction maléïmide du mini-CD4 activé avec le SMPH. La pureté finale (91%) du peptide GPR1 a été contrôlée par RP-HPLC analytique en utilisant un gradient linéaire de 20 à 40% (temps de rétention = 4,64 min).
ES⁺MS : :3376,44±0,49; attendu : 3376,58. Rendement : 12,9 mg (5,8 %).

### III.2 Couplage de GPR1 au mini-CD4 activé avec du SMPH

Un peptide GPR1 (1,5 mg ; 0,4 µmole) dans 200 µl de tampon phosphate pH = 7,4 a été ajouté à 200 µl d'une solution aqueuse de mini-CD4 activé avec du SMPH (0,95 mg ; 0,3 µmole). La réaction a été contrôlée par HPLC. Après 15 min, le pic correspondant au mini-CD4 activé avec le SMPH a complètement disparu. Le candidat peptide GPR1-mini-CD4 a été isolé sur une colonne Nucleosil C18 semi-préparative pour HPLC en phase inverse, 5 µm, 300 Å (10 X 250 mm) en utilisant un gradient linéaire de 35 à 55 % de CH₃CN dans du TFA aqueux à 0,08 %, sur une période de 20 min, à un débit de 6 ml/min.

La pureté finale (94,2 %) du peptide GPR1-mini-CD4 a été contrôlée par RP-HPLC analytique en utilisant un gradient linéaire de 35 à 55 % (temps de rétention = 12,60 min).
ES⁺MS : 6537,26 ± 0,54; attendu : 6537,38. Rendement : 1,9 mg (96 %).

### Exemple IV Pertinence du choix de la séquence générale (I) comportant un et un seul résidu lysine en position définie

La pertinence du choix de la séquence générale (I) comportant un et un seul résidu lysine en position définie a été validée par la synthèse d'un peptide miniCD4 dérivatisé sur la Lys par un (PEO)4-Biotin à l'aide du réactif EZ-Link-NHS-(PEO)4-Biotin PIERCE (Rockford, IL). L'introduction de ce dérivé Biotin en position définie dans la séquence générale (I) ne modifie pas la fixation de la gp120 sur le miniCD4 (mesure Biacore effectuée).

Les différentes synthèses n'ont pas été optimisées. De meilleurs rendements devraient être obtenus.

### Exemple V Synthèse chimique de mini-CD4 activés maléïmide via un espaceur PEO₂.

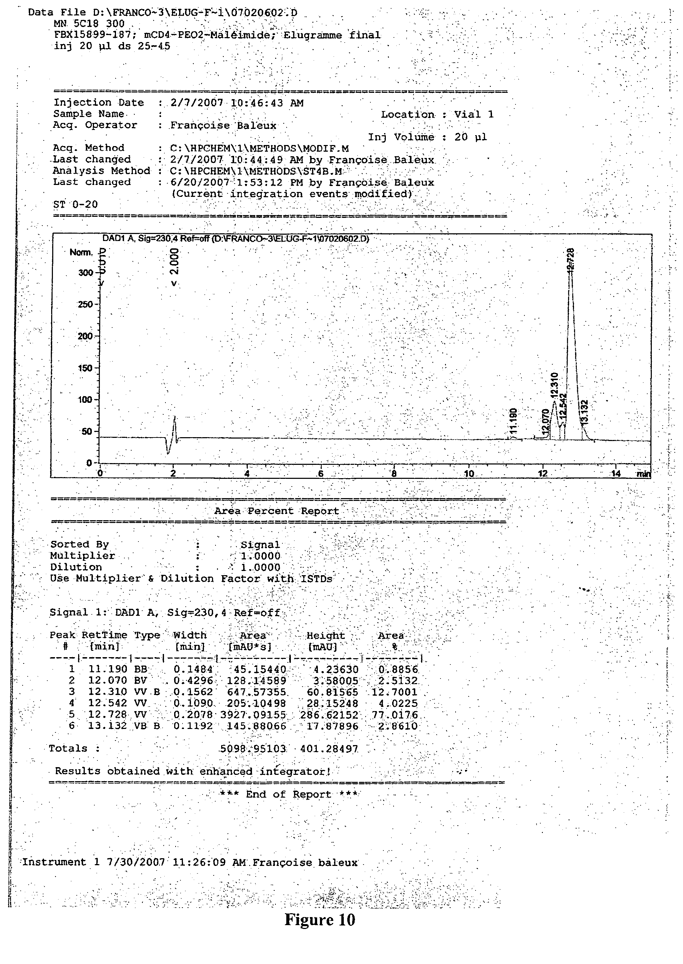

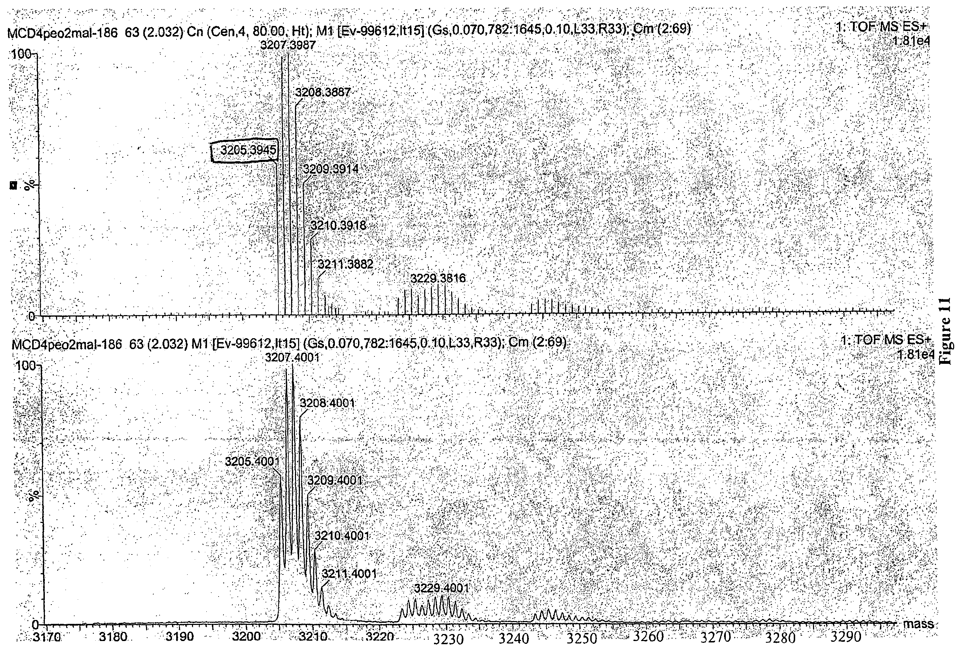

### Voir Figure 8, Figure 10 et Figure 11

Le mCD4- PEO₂-maléïmid diffère du composé mCD4-SMPH par la nature du bras espaceur (linker). En effet pour des raisons de solubilité, un linker polyéthylèneoxyde (PEO₂) plus hydrophile a été introduit entre le miniCD4 et la fonction maléïmide.

Synthèse : Une solution de 10 mg de mCD4 (MW : 2897 ; 3,4 mmoles) dans 1 ml H₂O a été diluée dans 1 ml de tampon phosphate de sodium 0,1 M pH8. A cette solution trouble ont été ajoutés 4,5 mg de NHS-PEO₂-Maléïmide (MW : 325, 13,8 mmoles ; 4 équiv.) dans 20 µl de DMSO sous agitation. Après 10 min, 85 % (HPLC) du matériel de départ a été converti en dérivé maléïmide. En raison de la faible stabilité du groupe maléïmide à pH8, la réaction de couplage a été directement chargée sur une colonne C18 SepaK équilibrée avec 10 % de CH3CN dans du TFA 0,08% aqueux. Le dérivé maléïmide a été élué avec 50% de CH3CN. Après lyophilisation, le composé a été ensuite purifié sur une colonne semi-préparative. Rendement: 5,2 mg (48 %); Pureté finale: 77%
ES+ : 3205,3938 ( M monoisotopique attendue: 3205,4211), QTOF micro Waters, MaxEnt1
Conditions HPLC:
Analytique: Nucleosil 5C18 300Å (4,6 x 15.0 mm); gradient linéaire 25-45 % de CH3CN dans du TFA 0,08% aqueux en 20 min à un débit de 1 ml/min. Détection:
230 nm. mCD4 Rt= 10,7 min; mCD4-PEO2-Mal Rt=12,8 min
Semi-préprative: Nucleosil 5C18 300Å (10 x 250 mm) ); gradient linéaire 25-45 % de CH3CN dans du TFA 0,08% aqueux en 20 min à un débit, de 6 ml/min.
Détection: 230 nm. mCD4-PEO2-Mal Rt=11,4 min

### Abréviations

- Fmoc :: 9-fluorénylméthyloxycarbonyle
- HATU :: N-oxyde d'hexafluorophosphate de N[(diméthylamino)-1H-1,2,3- triazolo [4,5-b]pyridin-1-ylméthylène]-N-méthylméthanaminium
- DIEA :: diisopropyléthylamine
- SPDP :: N-succinimidyl-3-(2-pyridyldithio)propionate
- TFA :: acide trifluoroacétique
- EDT :: éthanedithiol
- TIS :: triisopropylsilane
- DTT :: 1,4-dithiothréitol
- MPLC:: chromatographie liquide moyenne pression
- ES⁺MS :: spectrométrie de masse électrospray, en mode positif
- GSH :: glutathion réduit
- GSSG :: glutathion oxydé
- HPLC :: chromatographie liquide haute performance
- SMPH :: succinimidyl-6-[β-maléimidopropionamido]hexanoate
- SATP :: N-succinimidyl-S-acétylthiopropionate
- GPR1 :: récepteur 1 couplé à une protéine G

## Revendications

1. Procédé de préparation d'un peptide activé dérivé du récepteur CD4, ledit peptide, une fois activé, étant capable de se coupler par liaison covalente à une molécule organique, et dans lequel ledit peptide dérivé du récepteur CD4 comprend la séquence générale (I) suivante :
Xaa^{f} - P1 - Lys - Cys - P2 - Cys - P3 - Cys - Xaa^{g} - Xaa^{h} - Xaaⁱ - Xaa^{j} - Cys - Xaa^{k} - Cys - Xaa^{l} - Xaa^{m}, (I)
dans laquelle :
- P1 représente 3 à 6 résidus d'acides aminés,
- P2 représente 2 à 4 résidus d'acides aminés,
- P3 représente 6 à 10 résidus d'acides aminés,
- Xaa^{f} représente la N-acétylcystéïne (Ac-Cys) ou l'acide thiopropionique (TPA),
- Xaa^{g} représente Ala ou Gln,
- Xaa^{h} représente Gly ou (D)Asp ou Ser,
- Xaaⁱ représente Ser ou His ou Asn,
- Xaa^{j} représente la biphénylalanine (Bip), la phénylalanine ou la [bêta]-naphthylalanine,
- Xaa^{k} représente Thr ou Ala, et
- Xaa^{l} représente Gly, Val ou Leu,
- Xaa^{m} représente -NH₂ ou -OH,
les résidus d'acides aminés dans P1, P2 et P3 étant naturels ou non naturels, identiques ou différents, lesdits résidus de P1, P2 et P3 étant tous différents du résidu Lys, et P1, P2 et P3 ayant ou non une séquence commune,
**caractérisé en ce que** le procédé comprend la mise en présence du peptide de séquence générale (I) dérivé du récepteur CD4 avec un composé bifonctionnel portant deux groupes actifs, dont l'un au moins des deux groupes actifs est capable de former une liaison covalente avec la fonction amine libre (-NH₂) du résidu d'acide aminé Lys présent dans la séquence générale (I).

2. Procédé de préparation d'un peptide activé selon la revendication 1, dans lequel la séquence du peptide dérivé du récepteur CD4 de séquence générale (I) est choisie dans le groupe constitué par les séquences SEQ ID N°1 et SEQ ID N°2.

3. Procédé de préparation d'un peptide activé selon la revendication 1 ou 2, dans lequel le groupe actif capable de former une liaison covalente avec la fonction amine libre (-NH₂) du résidu d'acide aminé Lys présent dans la séquence générale (I), est le groupe actif N-hydroxysuccinimide ester (NHS).

4. Procédé de préparation d'un peptide activé selon la revendication 3, dans lequel les deux groupes actifs du composé bifonctionnel sont différents, et dans lequel l'un des deux groupes représente le groupe actif NHS.

5. Procédé de préparation d'un peptide activé selon la revendication 4, dans lequel le composé bifonctionnel est le succinimidyl-6-[bêta-maléimidopropionamido]hexanoate (SMPH).

6. Procédé de préparation d'un peptide activé selon la revendication 4, dans lequel le composé bifonctionnel est choisi dans le groupe constitué par le N-succinimidyl-S-acétylthioacétate (SATA) et le N-succinimidyl-S-acétylthiopropionate (SATP).

7. Procédé de préparation d'un peptide activé selon la revendication 4, dans lequel le composé bifonctionnel est NHS-PEOₙ-maléïmide, n étant compris entre 2 et 24.

8. Procédé de préparation d'un peptide activé selon la revendication 7 dans lequel n=2, le composé bifonctionnel étant l'ester de succinimidyl-[(N-maléïmidopropionamido)-diéthylèneglycol].

9. Procédé de préparation d'un peptide activé selon l'une des revendications 1 à 8 dans lequel Xaa^{f} représente TPA, comprenant une étape préliminaire de préparation du peptide dérivé du récepteur CD4 de séquence générale (I), dans laquelle le peptide dérivé du récepteur CD4 de séquence générale (II) suivante :
P1-Lys-Cys-P2-Cys-P3-Cys-Xaa^{g}-Xaa^{h}-Xaaⁱ - Xaa^{j} - Cys-Xaa^{k} -Cys-Xaa^{l}-Xaa^{m}, (II)
dans laquelle P1 à P3 et Xaa^{g} à Xaa^{m} sont tels que définis dans la séquence générale (I),
est mis en présence avec le N-succinimidyl 3-(2-pyridyldithio)propionate (SPDP) pour incorporer le TPA à l'extrémité N-terminale dudit peptide dérivé du récepteur CD4 de séquence générale (II).

10. Peptide activé dérivé du récepteur CD4 comprenant la séquence générale (I) telle que définie dans la revendication 1, dans lequel le résidu d'acide aminé Lys est lié de façon covalente, avantageusement par une liaison amine, à un groupe actif capable de se coupler par liaison covalente à une molécule organique.

11. Peptide activé selon la revendication 10, dans lequel le groupe actif est le groupe maléïmide.

12. Peptide activé selon la revendication 11, de structure moléculaire suivante :

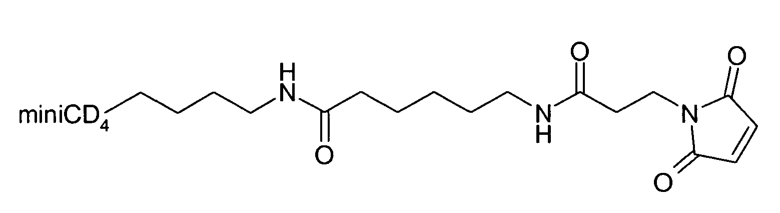

13. Peptide activé selon la revendication 11, de structure moléculaire suivante:

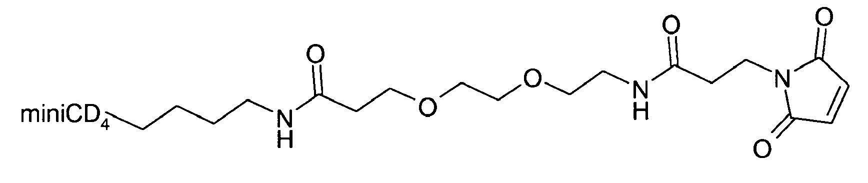

14. Peptide activé selon la revendication 10, dans lequel le groupe actif est le groupe thioacétyle.

15. Peptide activé selon la revendication 14, de structure moléculaire suivante:

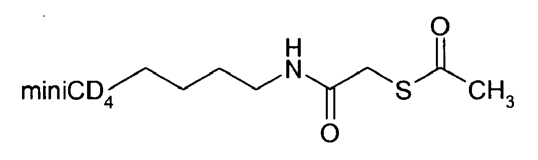

16. Peptide activé selon la revendication 14, de structure moléculaire suivante:

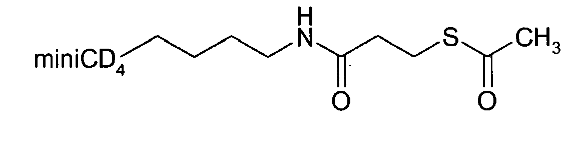

17. Procédé de préparation d'une molécule conjuguée comportant un peptide dérivé du récepteur CD4 couplé par liaison covalente à une molécule organique, ledit peptide dérivé du récepteur CD4 comprenant la séquence générale (I) telle que définie dans la revendication 1,
**caractérisé en ce que** le procédé comprend la mise en présence du peptide activé selon l'une des revendications 10 à 16 avec la molécule organique.

18. Procédé de préparation d'une molécule conjuguée selon la revendication 17, dans lequel le peptide activé est le peptide selon l'une des revendications 11 à 13 et la molécule organique porte une fonction thiol ou thioacétyle.

19. Procédé de préparation d'une molécule conjuguée selon la revendication 18, dans lequel la molécule organique portant une fonction thiol est le peptide GPR1 de séquence choisie dans le groupe constitué par les séquences SEQ ID N°3 et SEQ ID N°4.

20. Procédé de préparation d'une molécule conjuguée selon la revendication 18, dans lequel la molécule organique portant une fonction thiol est choisie dans le groupe constitué par les peptides comportant essentiellement des résidus acides, les peptides comportant essentiellement des résidus phosphorylables, et les peptides comportant essentiellement des résidus sulfatables.

21. Procédé de préparation d'une molécule conjuguée selon la revendication 18, dans lequel la molécule organique portant une fonction thiol est un polyanion modifié portant la fonction thiol.

22. Procédé de préparation d'une molécule conjuguée selon la revendication 21, dans lequel le polyanion modifié portant la fonction thiol est choisi dans le groupe constitué par l'héparine et l'héparane, sulfate et a un degré de polymérisation dp de 10 à 24.

23. Procédé de préparation d'une molécule conjuguée selon la revendication 17, dans lequel le peptide activé est le peptide selon l'une des revendications 14 à 16 et la molécule organique porte une fonction maléïmide ou halogène.

24. Molécule conjuguée comportant un peptide dérivé du récepteur CD4 comprenant la séquence générale (I) telle que définie dans la revendication 1 couplé à une molécule organique, dans laquelle le peptide dérivé du récepteur CD4 et la molécule organique sont couplés l'un à l'autre par un bras espaceur, et dans laquelle le résidu d'acide aminé Lys de la séquence générale (I) forme une liaison amine avec le bras espaceur.

25. Molécule conjuguée selon la revendication 24, dans laquelle la séquence générale (I) est la séquence SEQ ID N°1, et la molécule organique portant une fonction thiol est le peptide GPR1 de séquence choisie dans le groupe constitué par les séquences SEQ ID N°3 et SEQ ID N°4.

26. Molécule conjuguée selon la revendication 24, dans laquelle la séquence générale (I) est la séquence SEQ ID N°1, et la molécule organique portant une fonction thiol est le polyanion modifié portant la fonction thiol.

27. Utilisation du peptide activé selon l'une des revendications 11 à 13, pour le couplage d'une molécule organique comportant une fonction thiol ou thioacétyle.

28. Utilisation du peptide activé selon l'une des revendications 14 à 16, pour le couplage d'une molécule organique comportant une fonction maléïmide ou halogène.

29. Utilisation du peptide activé selon l'une des revendications 10 à 16, pour la fabrication d'un médicament destiné à un traitement antiviral.

30. Utilisation selon la revendication 29, dans laquelle le médicament est destiné à traiter le sida.

31. Molécule conjuguée selon la revendication 25 ou 26, pour son utilisation en tant que médicament.

32. Utilisation de la molécule conjuguée selon la revendication 25 ou 26 pour la fabrication d'un médicament destiné au traitement du sida.

## Claims

1. A method for preparing an activated peptide derived from the CD4 receptor, wherein the aforesaid peptide, once activated, is capable of coupling by covalent bonding to an organic molecule, and wherein the aforesaid peptide derived from the CD4 receptor comprises the following general sequence (I):
Xaa^{f}-P1-Lys-Cys-P2-Cys-P3-Cys-Xaa^{g}-Xaa ^{h}-Xaaⁱ-Xaa^{j}-Cys-Xaa^{k}-Cys-Xaa^{l}-Xaa^{m}, (I)
wherein:
- P1 represents 3 to 6 amino acid residues,
- P2 represents 2 to 4 amino acid residues,
- P3 represents 6 to 10 amino acid residues,
- Xaa^{f} represents N-acetylcysteine (Ac-Cys) or thiopropionic acid (TPA),
- Xaa^{g} represents Ala or Gln,
- Xaa^{h} represents Gly or (D)Asp or Ser,
- Xaaⁱ represents Ser or His or Asn,
- Xaa^{j} represents biphenylalanine (Bip), phenylalanine or [beta]-naphthylalanine,
- Xaa^{k} represents Thr or Ala, and
- Xaa^{l} represents Gly, Val or Leu,
- Xaa^{m} represents -NH₂ or -OH,
wherein the amino acid residues in P1, P2 and P3 are natural or not natural, identical or different, and wherein the aforesaid residues of P1, P2 and P3 are all different from the Lys residue, and wherein P1, P2 and P3 have or do not have a common sequence,
**characterized in that** the process comprises the bringing together of the peptide of general sequence (I) derived from the CD4 receptor with a bifunctional compound bearing two active groups, of which one at least of the two active groups is capable of forming a covalent bond with the free amine function (-NH₂) of the Lys amino acid residue present in the general sequence (I).

2. The method for preparing an activated peptide according to claim 1, wherein the sequence of the peptide derived from the CD4 receptor of general sequence (I) is selected from the group consisting of sequences SEQ ID NO 1 and SEQ ID NO 2.

3. The method for preparing an activated peptide according to claim 1 or 2, wherein the active group capable of forming a covalent bond with the free amine function (-NH₂) of the Lys amino acid residue present in the general sequence (I) is the active group N-hydroxysuccinimide ester (NHS).

4. The method for preparing an activated peptide according to claim 3, wherein the two active groups of the bifunctional compound are different, and wherein one of the two groups represents the active group NHS.

5. The method for preparing an activated peptide according to claim 4, wherein the bifunctional compound is succinimidyl-6-[beta-maleimidopropionamido] hexanoate (SMPH).

6. The method for preparing an activated peptide according to claim 4, wherein the bifunctional compound is selected from the group consisting of N-succinimidyl-S-acetylthioacetate (SATA) and N-succinimidyl-S-acetylthiopropionate (SATP).

7. The method for preparing an activated peptide according to claim 4, wherein the bifunctional compound is NHS-PEOₙ-maleimide, with n between 2 and 24.

8. The method for preparing an activated peptide according to claim 7, wherein n=2 and wherein the bifunctional compound is succinimidyl-[(N-maleimidopropionamido)-diethyleneglycol] ester.

9. The method for preparing an activated peptide according to one of claims 1 to 8, wherein Xaa^{f} represents TPA, and comprising a preliminary step of preparing the peptide derived from the CD4 receptor of general sequence (I), wherein the peptide derived from the CD4 receptor of following general sequence (II):
P1-Lys-Cys-P2-Cys-P3-Cys-Xaa^{g}-Xaa^{h}-Xaaⁱ-Xaa^{j}-Cys-Xaa^{k}-Cys-Xaa^{l}-Xaa^{m}, (II)
wherein P1 to P3 and Xaa^{g} to Xaa^{m} are such as defined in the general sequence (I),
is brought together with N-succinimidyl 3-(2-pyridyldithio)propionate (SPDP) to incorporate the TPA at the N-terminal end of the aforesaid peptide derived from the CD4 receptor of general sequence (II).

10. An activated peptide derived from the CD4 receptor comprising the general sequence (I) as defined in claim 1, wherein the Lys amino acid residue is bound covalently, advantageously by an amine bond, to an active group capable of coupling by covalent bonding to an organic molecule.

11. The activated peptide according to claim 10, wherein the active group is the maleimide group.

12. The activated peptide according to claim 11, of the following molecular structure:

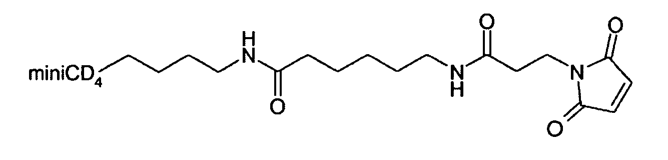

13. The activated peptide according to claim 11, of the following molecular structure:

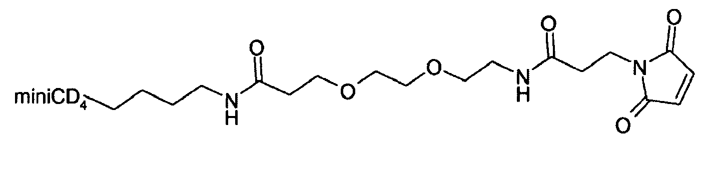

14. The activated peptide according to claim 10, wherein the active group is the thioacetyl group.

15. The activated peptide according to claim 14, of the following molecular structure:

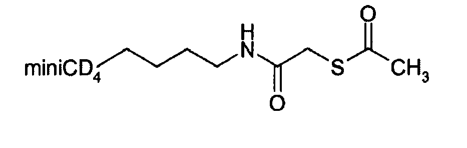

16. The activated peptide according to claim 14, of the following molecular structure:

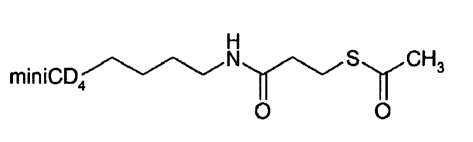

17. A method for preparing a conjugated molecule comprising a peptide derived from the CD4 receptor coupled by covalent bonding to an organic molecule, wherein the aforesaid peptide derived from the CD4 receptor comprises the general sequence (I) as defined in claim 1, **characterized in that** the method comprises the bringing together of the activated peptide according to one of claims 10 to 16 with the organic molecule.

18. The method for preparing a conjugated molecule according to claim 17, wherein the activated peptide is the peptide according to one of claims 11 to 13 and wherein the organic molecule bears a thiol or thioacetyl function.

19. The method for preparing a conjugated molecule according to claim 18, wherein the organic molecule bearing a thiol function is the peptide GPR1 of the sequence chosen from the group consisting of sequences SEQ ID NO 3 and SEQ ID NO 4.

20. The method for preparing a conjugated molecule according to claim 18, wherein the organic molecule bearing a thiol function is selected from the group consisting of peptides comprising essentially acid residues, peptides comprising essentially phosphorylatable residues, and peptides comprising essentially sulfatable residues.

21. The method for preparing a conjugated molecule according to claim 18, wherein the organic molecule bearing a thiol function is a modified polyanion bearing the thiol function.

22. The method for preparing a conjugated molecule according to claim 21, wherein the modified polyanion bearing the thiol function is selected from the group consisting of heparin and heparan sulfate and has a degree of polymerization (DP) of 10 to 24.

23. The method for preparing a conjugated molecule according to claim 17, wherein the activated peptide is the peptide according to one of claims 14 to 16 and the organic molecule bears a maleimide or halogen function.

24. A conjugated molecule comprising a peptide derived from the CD4 receptor comprising the general sequence (I) as defined in claim 1 coupled to an organic molecule, wherein the peptide derived from the CD4 receptor and the organic molecule are coupled together by a spacer arm, and wherein the Lys amino acid residue of the general sequence (I) forms an amine bond with the spacer arm.

25. The conjugated molecule according to claim 24, wherein the general sequence (I) is sequence SEQ ID NO 1, and the organic molecule bearing a thiol function is the peptide GPR1 of the sequence chosen from the group consisting of sequences SEQ ID NO 3 and SEQ ID NO 4.

26. The conjugated molecule according to claim 24, wherein the general sequence (I) is sequence SEQ ID NO 1, and the organic molecule bearing a thiol function is the modified polyanion bearing the thiol function.

27. Use of the activated peptide according to one of claims 11 to 13, for the coupling of an organic molecule comprising a thiol or thioacetyl function.

28. Use of the activated peptide according to one of claims 14 to 16, for the coupling of an organic molecule comprising a maleimide or halogen function.

29. Use of the activated peptide according to one of claims 10 to 16, for the manufacture of a drug intended for an antiviral treatment.

30. The use according to claim 29, wherein the drug is intended to treat AIDS.

31. The conjugated molecule according to claim 25 or 26, for its use as a drug.

32. Use of the conjugated molecule according to claim 25 or 26, for the manufacture of a drug intended for the treatment of AIDS.

## Patentansprüche

1. Verfahren zur Herstellung eines aktivierten Peptids, das von dem CD4-Rezeptor abgeleitet ist, wobei das Peptid, sobald es aktiviert ist, in der Lage ist, sich durch kovalente Bindung an ein organisches Molekül zu binden, und in welchem das von dem CD4-Rezeptor abgeleitete Peptide die folgende allgemeine Sequenz (I) umfasst:
Xaa^{f} - P1 - Lys - Cys - P2 - Cys - P3 - Cys - Xaa^{g} - Xaa^{h} - Xaaⁱ - Xaa^{j} - Cys - Xaa^{k} - Cys - Xaa^{l} - Xaa^{m} (I),
in welcher:
- P1 für 3 bis 6 Aminosäurereste steht,
- P2 für 2 bis 4 Aminosäurereste steht,
- P3 für 6 bis 10 Aminosäurereste steht,
- Xaa^{f} für N-Acetylcystein (Ac-Cys) oder Thiopropionsäure (TPA) steht,
- Xaa^{g} für Ala oder Gln steht,
- Xaa^{h} für Gly oder (D)Asp oder Ser steht,
- Xaaⁱ für Ser oder His oder Asn steht;
- Xaa^{j} für Biphenylalanin (Bip), Phenylalanin oder beta-Naphthylalanin steht,
- Xaa^{k} für Thr oder Ala steht und
- Xaa^{l} für Gly, Val oder Leu steht,
- Xaa^{m} für -NH₂ oder -OH steht,
wobei die Aminosäurereste in P1, P2 und P3 natürlich oder nicht-natürlich, gleich oder verschieden sind, wobei die Reste von P1, P2 und P3 allesamt von dem Rest Lys verschieden sind und P1, P2 und P3 eine gemeinsame Sequenz aufweisen oder nicht,
**dadurch gekennzeichnet, dass** das Verfahren umfasst, das von dem CD4-Rezeptor abgeleitete Peptid der allgemeinen Sequenz (I) mit einer bifunktionellen Verbindung, die zwei aktive Gruppen trägt, von welcher mindestens eine der zwei aktiven Gruppen in der Lage ist, eine kovalente Bindung mit der freien Aminfunktion (-NH₂) des in der allgemeinen Sequenz (I) vorhandenen Aminosäurerests Lys zu bilden, in Kontakt zu bringen.

2. Verfahren zur Herstellung eines aktivierten Peptids nach Anspruch 1, in welchem die Sequenz des von dem CD4-Rezeptor abgeleiteten Peptids der allgemeinen Sequenz (I) in der Gruppe, die aus den Sequenzen SEQ ID No. 1 und SEQ ID No. 2 gebildet wird, ausgewählt ist.

3. Verfahren zur Herstellung eines aktivierten Peptids nach Anspruch 1 oder 2, in welchem die aktive Gruppe, die in der Lage ist, eine kovalente Bindung mit der freien Aminfunktion (-NH₂) des in der allgemeinen Sequenz (I) vorhandenen Aminosäurerests Lys zu bilden, die aktive N-Hydroxysuccinimidester-Gruppe (NHS) ist.

4. Verfahren zur Herstellung eines aktivierten Peptids nach Anspruch 3, in welchem die beiden aktiven Gruppen der bifunktionellen Verbindung unterschiedlich sind und in welchem eine der beiden Gruppen für die aktive NHS-Gruppe steht.

5. Verfahren zur Herstellung eines aktivierten Peptids nach Anspruch 4, in welchem die bifunktionelle Verbindung Succinimidyl-6-[beta-maleimidopropionamido]hexanoat (SMPH) ist.

6. Verfahren zur Herstellung eines aktivierten Peptids nach Anspruch 4, in welchem die bifunktionelle Verbindung in der aus N-Succinimidyl-S-acetylthioacetat (SATA) und N-Succinimidyl-S-acetylthiopropionat (SATP) gebildeten Gruppe ausgewählt ist.

7. Verfahren zur Herstellung eines aktivierten Peptids nach Anspruch 4, in welchem die bifunktionelle Verbindung NHS-PEGₙ-Maleimid ist, wobei n zwischen 2 und 24 eingeschlossen liegt.

8. Verfahren zur Herstellung eines aktivierten Peptids nach Anspruch 7, in welchem n = 2, wobei die bifunktionelle Verbindung der Ester von Succinimidyl-[(N-maleimidopropionamido)-diethylenglycol] ist.

9. Verfahren zur Herstellung eines aktivierten Peptids nach einem der Ansprüche 1 bis 8, in welchem Xaa^{f} für TPA steht, welches einen vorab erfolgenden Schritt einer Herstellung des von dem CD4-Rezeptor abgeleiteten Peptids der allgemeinen Sequenz (I) umfasst, bei welcher das von dem CD4-Rezeptor abgeleitete Peptid der folgenden allgemeinen Sequenz (II):
P1 - Lys - Cys - P2 - Cys - P3 - Cys - Xaa^{g} - Xaa^{h} - Xaaⁱ - Xaa^{j} - Cys - Xaa^{k} - Cys - Xaa^{l} - Xaa^{m} (II),
in welcher P1 bis P3 und Xaa^{g} bis Xaa^{m} wie in der allgemeinen Sequenz (I) definiert sind,
mit N-Succinimidyl-3-(2-pyridyldithio)propionat (SPDP) in Kontakt gebracht wird, um das TPA an das N-terminale Ende des von dem CD4-Rezeptor abgeleiteten Peptids der allgemeinen Sequenz (II) anzufügen.

10. Von dem CD4-Rezeptor abgeleitetes aktiviertes Peptid, welches die allgemeine Sequenz (I) wie in Anspruch 1 definiert umfasst, in welchem der Aminosäurerest Lys auf kovalente Weise, in vorteilhafter Weise durch eine Aminbindung, mit einer aktiven Gruppe, die in der Lage ist, sich durch kovalente Bindung an ein organisches Molekül zu binden, verknüpft ist.

11. Aktiviertes Peptid nach Anspruch 10, in welchem die aktive Gruppe die Maleimid-Gruppe ist.

12. Aktiviertes Peptid nach Anspruch 11 mit der folgenden molekularen Struktur:

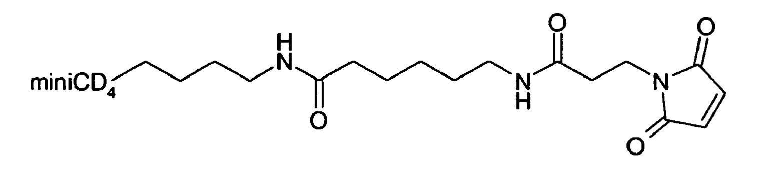

13. Aktiviertes Peptid nach Anspruch 11 mit der folgenden molekularen Struktur:

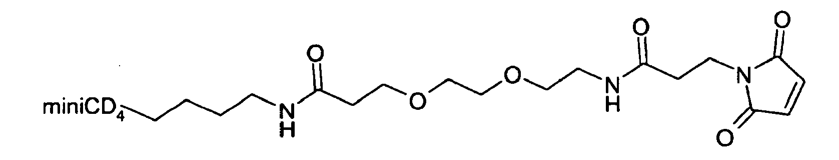

14. Aktiviertes Peptid nach Anspruch 10, in welchem die aktive Gruppe die Thioacetyl-Gruppe ist.

15. Aktiviertes Peptid nach Anspruch 14 mit der folgenden molekularen Struktur:

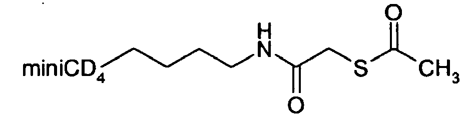

16. Aktiviertes Peptid nach Anspruch 14 mit der folgenden molekularen Struktur:

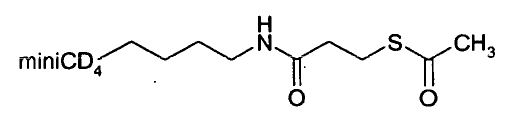

17. Verfahren zur Herstellung eines konjugierten Moleküls, welches ein von dem CD4-Rezeptor abgeleitetes Peptid, das durch kovalente Bindung an ein organisches Molekül gebunden ist, umfasst, wobei das von dem CD4-Rezeptor abgeleitete Peptid die allgemeine Sequenz (I) wie in Anspruch 1 definiert umfasst, **dadurch gekennzeichnet, dass** das Verfahren umfasst, das aktivierte Peptid nach einem der Ansprüche 10 bis 16 mit dem organischen Molekül in Kontakt zu bringen.

18. Verfahren zur Herstellung eines konjugierten Moleküls nach Anspruch 17, in welchem das aktivierte Peptid das Peptid nach einem der Ansprüche 11 bis 13 ist und das organische Molekül eine Thiol- oder Thioacetyl-Funktion trägt.

19. Verfahren zur Herstellung eines konjugierten Moleküls nach Anspruch 18, in welchem das organische Molekül, das eine Thiol-Funktion trägt, das Peptid GPRI mit einer Sequenz, die in der aus den Sequenzen SEQ ID No. 3 und SEQ ID No. 4 gebildeten Gruppe ausgewählt ist, ist.

20. Verfahren zur Herstellung eines konjugierten Moleküls nach Anspruch 18, in welchem das organische Molekül, das eine Thiol-Funktion trägt, in der Gruppe, die aus den Peptiden, die im Wesentlichen saure Reste umfassen, den Peptiden, die im Wesentlichen phosphorylierbare Reste umfassen, und den Peptiden, die im Wesentlichen sulfatierbare Reste umfassen, gebildet wird, ausgewählt ist.

21. Verfahren zur Herstellung eines konjugierten Moleküls nach Anspruch 18, in welchem das organische Molekül, das eine Thiol-Funktion trägt, ein modifiziertes Polyanion, welches die Thiol-Funktion trägt, ist.

22. Verfahren zur Herstellung eines konjugierten Moleküls nach Anspruch 21, in welchem das modifizierte Polyanion, welches die Thiol-Funktion trägt, in der aus Heparin und Heparansulfat gebildeten Gruppe ausgewählt ist und einen Polymerisationsgrad Pg von 10 bis 24 aufweist.

23. Verfahren zur Herstellung eines konjugierten Moleküls nach Anspruch 17, in welchem das aktivierte Peptid das Peptid nach einem der Ansprüche 14 bis 16 ist und das organische Molekül eine Maleimid- oder Halogenfunktion trägt.

24. Konjugiertes Molekül, das ein von dem CD4-Rezeptor abgeleitetes Peptid, umfassend die allgemeine Sequenz (I) wie in Anspruch 1 definiert, gebunden an ein organisches Molekül umfasst, in welchem das von dem CD4-Rezeptor abgeleitete Peptid und das organische Molekül durch einen Spacer-Arm aneinander gebunden sind und in welchem der Aminosäurerest Lys der allgemeinen Sequenz (I) eine Aminbindung mit dem Spacer-Arm bildet.

25. Konjugiertes Molekül nach Anspruch 24, in welchem die allgemeine Sequenz (I) die Sequenz SEQ ID No. 1 ist und das organische Molekül, das eine Thiol-Funktion trägt, das Peptid GPRI mit einer Sequenz, die in der aus den Sequenzen SEQ ID No. 3 und SEQ ID No. 4 gebildeten Gruppe ausgewählt ist, ist.

26. Konjugiertes Molekül nach Anspruch 24, in welchem die allgemeine Sequenz (I) die Sequenz SEQ ID No. 1 ist und das organische Molekül, das eine Thiol-Funktion trägt, das modifizierte Polyanion, das die Thiol-Funktion trägt, ist.

27. Verwendung des aktivierten Peptids nach einem der Ansprüche 11 bis 13 für die Anheftung eines organischen Moleküls, das eine Thiol- oder Thioacetyl-Funktion umfasst.

28. Verwendung des aktivierten Peptids nach einem der Ansprüche 14 bis 16 für die Anheftung eines organischen Moleküls, das eine Maleimid- oder Halogen-Funktion umfasst.

29. Verwendung des aktivierten Peptids nach einem der Ansprüche 10 bis 16 für die Herstellung eines Arzneimittels, das für eine antivirale Behandlung bestimmt ist.

30. Verwendung nach Anspruch 29, in welchem das Arzneimittel für die Behandlung von AIDS bestimmt ist.

31. Konjugiertes Molekül nach Anspruch 25 oder 26 zur Verwendung als Arzneimittel.

32. Verwendung des konjugierten Moleküls nach Anspruch 25 oder 26 für die Herstellung eines Arzneimittels, das für die Behandlung von AIDS bestimmt ist.
